# EUROPEAN PATENT APPLICATION

(11) **EP 4 537 907 A1**
(43) Date of publication of application: **16.04.2025**
(21) Application number: 23202742.5
(22) Date of filing: 10.10.2023
(51) Int. Cl.: A61P 43/00, C07K 16/28

(54) **CD248 INHIBITORS AND USES THEREOF**

(71) Applicant: Enthera S.r.l., 20122 Milano (MI) (IT)
(72) Inventor: AMABILE, Giovanni, 20122 Milano (IT); FIORINA, Paolo, 20122 Milano (IT); D'ADDIO, Francesca, 20122 Milano (IT); MARIN, Virna, 20122 Milano (IT); PORZIO, Stefano, 20122 Milano (IT)
(74) Representative: Pace Napoleone, Maria

(57) **Abstract**

The present invention relates to inhibitors of CD248, pharmaceutical compositions containing said inhibitors, and uses thereof. In particular, the invention relates to antibodies or antigen binding fragments thereof that bind specifically to human CD248 and have an anti-fibrotic effect.

## Description

### TECHNICAL FIELD

The present invention relates to inhibitors of CD248, methods for their production, pharmaceutical compositions containing said inhibitors, and uses thereof. In particular, the invention relates to antibodies or antigen binding fragments thereof that bind specifically to human CD248, wherein said antibodies have an anti-fibrotic effect.

### BACKGROUND ART

A number of diseases are characterized by pathogenic fibrosis.

Idiopathic pulmonary fibrosis (IPF) is a non-neoplastic chronic lung syndrome that is characterized by aberrant accumulation of fibroblasts/myofibroblasts and progressive abnormal remodeling of lung parenchyma, with subsequent scarring and disruption of its structure and function. Despite considerable recent progress, current medical treatment regimens are largely unpromising with a median survival rate of three to five years from the date of diagnosis (Glass et al. Clin Respir J. 2022. 16(2):84-96); lung transplant is today the final therapeutic option. Systemic sclerosis (SSc) is a rare systemic autoimmune connective tissue disease characterized by vasculopathy, immune dysregulation and progressive fibrosis, affecting primarily the skin, gastrointestinal tract, lungs, heart and kidneys (Henes et all. Haematologica. 2021. 106 (2): 375-383). It is associated with high disease related mortality and the main causes of death are related to cardiac, pulmonary and renal involvement. There are still no effective treatments to prevent or halt the progression of fibrosis in SSc (Bukiri et al. Curr Opin Pharmacol. 2022 Jun;64:102211.) Liver fibrosis (also called hepatic fibrosis) is a non-physiological irreversible scarring process that leads to tissue damage and failure/disturbance of liver function. If not treated, it may lead to advanced liver cirrhosis and hepatocellular carcinoma (HCC). Liver fibrosis and in particular cirrhosis are the major causes of morbidity and mortality of patients with chronic liver disease (GBD 2017 Cirrhosis Collaborators 2020. Lancet Gastroenterol Hepatol 2020; 5: 245-66). Kidney fibrosis (also called renal fibrosis) represents the end-stage of a progressive and irreversible renal disease in which the kidneys do not work properly. It is characterized by loss of renal cells mass and their replacement by the excessive deposition of the extracellular matrix (ECM) in glomeruli, vessels and interstitium that cause glomerulosclerosis, vascular sclerosis and tubulointerstitial fibrosis (António Nogueira et al. in vivo 31: 1-22 (2017)).

There is still the need in the art for treatments that are capable of reducing progression of fibrosis in fibrotic diseases.

### SUMMARY OF THE INVENTION

CD248, a glycosylated transmembrane protein, has previously been associated with various fibrotic diseases.

It was now found that inhibition of the binding of insulin-like growth factor binding protein 5 (IGFBP5) to CD248 reduces fibrosis in a model of fibrotic disease.

Identification of CD248 as a binding partner of IGFBP5 newly identifies the CD248:IGFBP5 interaction as an important target for fibrosis. Indeed, an entire pro-fibrotic pathway can be advantageously blocked while targeting a single molecule (CD248).

Many known anti-fibrotic drugs target only one single pro-fibrotic factor downstream the cascade. Instead, by targeting CD248/IGFBP5 axis it is possible to block the entire pathway with consequent downregulation of several pro-fibrotic factors and ECM genes. In particular, antibodies advantageously showing specificity for CD248 and inhibitory activity of the binding between human recombinant CD248 and human recombinant IGFBP5 have been identified.

Therefore, in a first aspect, inhibitors of the CD248 receptor that inhibit or reduce the binding of IGFBP5 to CD248 are provided. These inhibitors have anti-fibrotic activity and/or anti-fibrosis activity.

The anti-fibrotic effect may be measured by any means known in the art, including as described herein. The inhibitor is considered to have anti-fibrotic effect when fibrosis is inhibited by at least 10% when compared to a control condition without antibody or antigen binding fragment thereof. Preferably the inhibitor is considered to have anti-fibrotic effect when fibrosis is inhibited by at least 15, 20, 25, 30, 35, 40, 50, 60, 70, 80 or 90 % when compared to a control condition without antibody or antigen binding fragment thereof.

Preferably the inhibitor of CD248 and/or of at least one of its binding partners is for use in the treatment and/or prevention of fibrosis or of a fibrotic condition.

Still preferably said inhibitor is selected from the group consisting of:
a) a polypeptide;
b) a polynucleotide or a polynucleotide coding for said polypeptide;
c) a vector comprising or expressing said polynucleotide;
d) a host cell genetically engineered expressing said polypeptide or said polynucleotide;
e) a small molecule;
f) a peptide, a protein, an antibody, an antisense oligonucleotide, a siRNA, antisense expression vector or recombinant virus.

More preferably said inhibitor is an isolated antibody or antigen binding fragment thereof that binds to human CD248, wherein said isolated antibody or antigen binding fragment thereof has anti-fibrotic effect.

Preferably the inhibitor inhibits, reduces, or neutralizes the synthesis of fibronectin 1 and collagen 1 alpha 1 mRNA from human lung epithelial cells or human lung fibroblasts induced by contact with the combination of IGFBP5 and TGF-β1 and/or inhibits, reduces, or neutralizes the secretion of fibronectin (FN1) from lung epithelial cells induced by contact with the combination of IGFBP5 and TGF-β1.

Preferably the isolated antibody or antigen binding fragment thereof comprises:
a. a heavy chain variable domain (VH) comprising:
   i. a CDR1 sequence of the amino acid sequence selected from the group consisting of: SEQ ID NO: 1, 2, and 3;
   ii. a CDR2 sequence of the amino acid sequence selected from the group consisting of: SEQ ID NO: 4, 5 and 6; and
   iii. a CDR3 sequence of the amino acid sequence selected from the group consisting of: SEQ ID NO: 7, 8, 9, 10, 11 and 12; and/or
b. a light chain variable domain (VL) comprising:
   i. a CDR1 sequence of the amino acid sequence selected from the group consisting of: SEQ ID NO: 13, 14, 15, 16, 17 and 18;
   ii. a CDR2 sequence of the amino acid sequence selected from the group consisting of: SEQ ID NO: 19, 20, 21, 22 and 23; and
   iii. a CDR3 sequence of the amino acid sequence selected from the group consisting of: SEQ ID NO: 24, 25, 26, 27, 28 and 29.

Still preferably, the isolated antibody or antigen binding fragment thereof comprises VH CDR1, CDR2 and CDR3 selected from Table 4 and VL CDR1, CDR2 and CDR3 selected from Table 5.

Preferably the isolated antibody or antigen binding fragment thereof comprises as CDRs:
- SEQ ID NO: 1 and SEQ ID NO: 4 and SEQ ID NO: 7 and SEQ ID NO: 13 and SEQ ID NO: 19 and SEQ ID NO: 24 or Kabat, IMGT, Chothia, AbM, or Contact CDRs of YU1022-D08 or
- SEQ ID NO: 2 and SEQ ID NO: 5 and SEQ ID NO: 8 and SEQ ID NO: 14 and SEQ ID NO: 20 and SEQ ID NO: 25 or Kabat, IMGT, Chothia, AbM, or Contact CDRs of YU1023-A02 or
- SEQ ID NO: 1 and SEQ ID NO: 4 and SEQ ID NO: 9 and SEQ ID NO: 15 and SEQ ID NO: 21 and SEQ ID NO: 26 or Kabat, IMGT, Chothia, AbM, or Contact CDRs of YU1023-B03 or
- SEQ ID NO: 3 and SEQ ID NO: 6 and SEQ ID NO: 10 and SEQ ID NO: 16 and SEQ ID NO: 22 and SEQ ID NO: 27 or Kabat, IMGT, Chothia, AbM, or Contact CDRs of YU1023-E03 or
- SEQ ID NO: 3 and SEQ ID NO: 6 and SEQ ID NO: 11 and SEQ ID NO: 17 and SEQ ID NO: 23 and SEQ ID NO: 28 or Kabat, IMGT, Chothia, AbM, or Contact CDRs of YU1024-G01 or
- SEQ ID NO: 3 and SEQ ID NO: 6 and SEQ ID NO: 12 and SEQ ID NO: 18 and SEQ ID NO: 19 and SEQ ID NO: 29 or Kabat, IMGT, Chothia, AbM, or Contact CDRs of YU1025-B07.

Each of said CDRs may also vary of 1 or 2 amino acids so long as the antibody activity, in particular the CD248 inhibitory activity, is retained.

Still preferably, the isolated antibody or antigen binding fragment thereof comprises:
a. a heavy chain variable domain (VH) comprising:
   i. a CDR1 sequence of the amino acid sequence selected from the group consisting of a sequence as defined using abysis tool analysis (www.abysis.org) preferably as shown in Tables 8-13;
   ii. a CDR2 sequence of the amino acid sequence selected from the group consisting of a sequence as defined using abysis tool analysis (www.abysis.org) preferably as shown in Tables 8-13; and
   iii. a CDR3 sequence of the amino acid sequence selected from the group consisting of a sequence as defined using abysis tool analysis (www.abysis.org) preferably as shown in Tables 8-13; and/or
b. a light chain variable domain (VL) comprising:
   i. a CDR1 sequence of the amino acid sequence selected from the group consisting of a sequence as defined using abysis tool analysis (www.abysis.org) preferably as shown in Tables 8-13;
   ii. a CDR2 sequence of the amino acid sequence selected from the group consisting of a sequence as defined using abysis tool analysis (www.abysis.org) preferably as shown in Tables 8-13; and
   iii. a CDR3 sequence of the amino acid sequence selected from the group consisting of a sequence as defined using abysis tool analysis (www.abysis.org) preferably as shown in Tables 8-13.

Still preferably, the isolated antibody or antigen binding fragment thereof comprises:
a. a heavy chain variable domain sequence of the amino acid sequence selected from the group consisting of: SEQ ID NO: 30, 31, 32, 33, 34 and 35 or
b. a light chain variable domain sequence of the amino acid sequence selected from the group consisting of: SEQ ID NO: 36, 37, 38, 39, 40 and 41 or
c. the heavy chain variable domain of (a) and the light chain variable domain of (b).

Still preferably, the isolated antibody or antigen binding fragment thereof is selected from the group consisting of: YU1022-D08, YU1023-A02, YU1023-B03, YU1023-E03, YU1024-G01 and YU1025-B07, as defined in Tables 4-18, preferably the isolated antibody or antigen binding fragment thereof is the antibody YU1023-A02 or YU1022-D08.

Anti-fibrotic activity of said anti-CD248 antibodies was shown on epithelial lung cells derived from a human alveolar epithelial carcinoma cell line (AEC, A549) and human lung primary fibroblasts (HLF) derived from a control donor or from an IPF patient, which are recognized in vitro models to test anti-fibrotic activity.

Still preferably the isolated antibody is YU1022-D08 comprising a heavy chain variable domain comprising or consisting of a sequence of SEQ ID NO: 30 and a light chain variable domain comprising or consisting of a sequence of SEQ ID NO: 36, YU1023-A02 comprising a heavy chain variable domain comprising or consisting of a sequence of SEQ ID NO: 31 and a light chain variable domain comprising or consisting of a sequence of SEQ ID NO: 37, YU1023-B03 comprising a heavy chain variable domain comprising or consisting of a sequence of SEQ ID NO: 32 and a light chain variable domain comprising or consisting of a sequence of SEQ ID NO: 38, YU1023-E03 comprising a heavy chain variable domain comprising or consisting of a sequence of SEQ ID NO: 33 and a light chain variable domain comprising or consisting of a sequence of SEQ ID NO: 39, YU1024-G01 comprising a heavy chain variable domain comprising or consisting of a sequence of SEQ ID NO: 34 and a light chain variable domain comprising or consisting of a sequence of SEQ ID NO: 40, or YU1025-B07 comprising a heavy chain variable domain comprising or consisting of a sequence of SEQ ID NO: 35 and a light chain variable domain comprising or consisting of a sequence of SEQ ID NO: 41.

Preferably, the isolated antibody comprises a heavy chain variable domain encoded by a nucleotide sequence selected from the group consisting of: SEQ ID NO: 42, SEQ ID NO: 43, SEQ ID NO: 44, SEQ ID NO: 45, SEQ ID NO: 46 and SEQ ID NO: 47; and/or a light chain variable domain encoded by a nucleotide sequence selected from the group consisting of: SEQ ID NO:48, SEQ ID NO: 49, SEQ ID NO: 50, SEQ ID NO: 51, SEQ ID NO: 52 and SEQ ID NO:53.

Yet preferably, the isolated antibody or antigen binding fragment thereof has an affinity constant (K_{D}) lower than or equal to 3×10⁻⁸ M for human CD248.

It is also disclosed an isolated antibody or antigen binding fragment thereof that:
(a) binds specifically to an epitope on CD248, said epitope being the same or similar epitope as the epitope recognized by any of the monoclonal antibodies as defined in Tables 4-18; or
(b) cross-competes for binding with any of the monoclonal antibodies as defined in Tables 4-18; or
(c) shows the same or similar binding affinity or specificity, or both, as any of the monoclonal antibodies as defined in Tables 4-18; or
(d) has one or more biological properties of an antibody molecule described herein, e.g., an antibody molecule chosen from, e.g., any as defined in Tables 4-18 and/or
(e) has one or more pharmacokinetic properties of an antibody molecule described herein, e.g., an antibody molecule chosen from, e.g., any as defined in Tables 4-18. Preferably the isolated antibody or antigen binding fragment thereof as defined above is a human or a humanized antibody.

More preferably, the isolated antibody or antigen binding fragment thereof is an IgG1 or IgG2 or IgG3 or IgG4 antibody, preferably an IgG1 kappa antibody, an IgG1 lambda antibody, an IgG2 kappa antibody, an IgG2 lambda antibody, an IgG3 kappa antibody or an IgG3 lambda antibody, an IgG4 kappa antibody or an IgG4 lambda antibody, preferably said IgG1 or IgG2 or IgG3 or IgG4 is human IgG1 or human IgG2 or human IgG3 or human IgG4. In a preferred embodiment, it is an IgG2 or IgG4 antibody, preferably an IgG2 kappa antibody, an IgG2 lambda antibody, an IgG4 kappa antibody or an IgG4 lambda antibody.

An isolated antibody or antigen binding fragment thereof comprising:
a. a heavy chain variable domain sequence of the amino acid sequence selected from the group consisting of: SEQ ID NO: 30, 31, 32, 33, 34 and 35 or
b. a light chain variable domain sequence of the amino acid sequence selected from the group consisting of: SEQ ID NO: 36, 37, 38, 39, 40 and 41 or
c. the heavy chain variable domain of (a) and the light chain variable domain of (b), is also an object of the invention.

It is herein disclosed an isolated polynucleotide comprising at least one sequence that encodes the antibody or antigen binding fragment thereof as herein defined, wherein preferably said polynucleotide is a cDNA. Preferably, such polynucleotide comprises one or more sequences selected from the group consisting of SEQ ID NO.42 to SEQ ID NO.53, in particular as disclosed in Tables 16 and 17.

It is herein disclosed a vector comprising the polynucleotide as defined above, preferably said vector being selected from the group consisting of a plasmid, a viral vector, a non-episomal mammalian vector, an expression vector and a recombinant expression vector.

It is herein disclosed an isolated cell comprising the polynucleotide or the vector as defined above, preferably said isolated cell being a hybridoma or a Chinese Hamster Ovary (CHO) cell or a Human Embryonic Kidney cells (HEK293).

Preferably the inhibitor, the antibody or antigen binding fragment thereof, or the polynucleotide or the vector or the cell as defined above is for use as a medicament, preferably for use in the treatment and/or prevention of fibrosis or of a fibrotic condition and/or for reducing progression of fibrosis or of a fibrotic condition, preferably scleroderma or pulmonary fibrosis, preferably morphea, fibrosis as a result of Graft-Versus-Host Disease (GVHD), keloid and hypertrophic scar, subepithelial fibrosis, endomyocardial fibrosis, uterine fibrosis, myelofibrosis, retroperitoneal fibrosis, nephrogenic systemic fibrosis, scarring after surgery, asthma, cirrhosis/liver fibrosis, aberrant wound healing, glomerulonephritis, and multifocal fibrosclerosis, cryptogenic fibrosing alveolitis (CFA), idiopathic pulmonary fibrosis (IPF), liver fibrosis, kidney fibrosis, in a subject who has, or is at risk of developing, said fibrotic condition.

It is herein disclosed a pharmaceutical composition comprising the inhibitor, isolated antibody or antigen binding fragment thereof, or the polynucleotide or the vector or the cell as described above and at least one pharmaceutically acceptable carrier, preferably for use in the treatment of fibrosis or of a fibrotic condition. Preferably said composition further comprises a second therapeutic agent.

Preferably, said second therapeutic agent is selected from nintedanib, pirfenidone, pamrevlumab, galectin-3 inhibitors, autotaxin inhibitors, pentraxins, tocilizumab, rituximab and romilkimab.

The antibody or antigen-binding fragment thereof disclosed herein can be used, alone or in combination with other agents or therapeutic modalities, to treat and/or prevent fibrosis.

Additionally, disclosed herein are methods and compositions comprising a combination of the antibody or antigen-binding fragment thereof of the invention with a second agent that treats fibrosis.

Antibodies and antigen binding fragments thereof are disclosed herein that have anti-fibrotic activity.

In some embodiments, methods are disclosed for inhibiting fibrosis *in vivo* or *in vitro.* In additional embodiments, methods are disclosed for the treatment of fibrosis in a subject. In some specific non-limiting examples, the subject has scleroderma or pulmonary fibrosis.

The anti-CD248 antibody molecules disclosed herein and antigen-binding fragments thereof can inhibit, reduce or neutralize one or more activities of IGFBP5. Thus, such antibody molecules and antigen-binding fragments can be used to treat or prevent disorders where the inhibition, reduction or neutralization of IGFBP5-induced activities in a subject is desired.

The foregoing and other features and advantages will become more apparent from the following detailed description of several embodiments, which proceeds with reference to the accompanying figures.

### BRIEF DESCRIPTION OF THE DRAWINGS

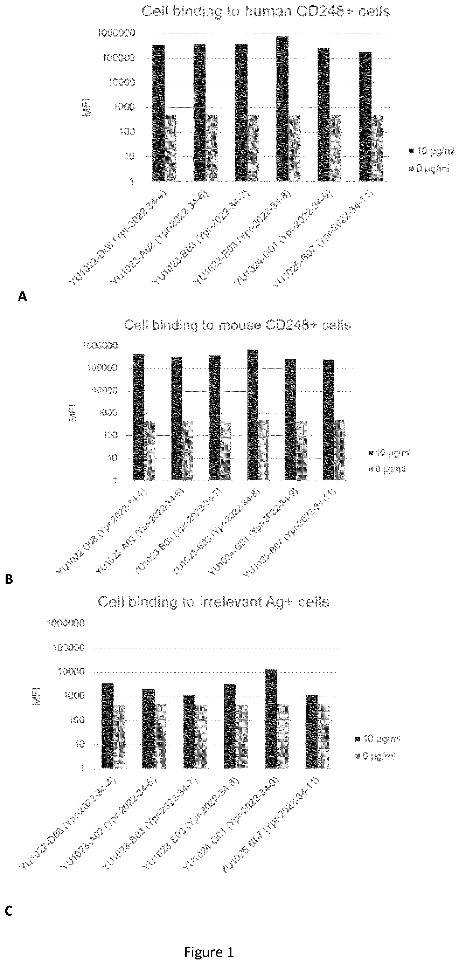

**Figures. 1A-1C** **are bar graphs summarizing binding** of anti-CD248 mAbs to cells respectively expressing human CD248, mouse CD248, and an irrelevant antigen. FIG. 1A shows data from anti-CD248 mAbs binding to human CD248 expressed on HEK 293 cells after transient transfection (human CD248⁺ cells). FIG. 1B shows data from anti-CD248 mAbs binding to mouse CD248 expressed on HEK 293 cells after transient transfection (mouse CD248⁺ cells). FIG. 1C shows data from anti-CD248 mAbs binding to an irrelevant antigen expressed on HEK cells after transient transfection (irrelevant Ag+ cells). Cells were incubated with and without anti-CD248 mAbs, i.e., 10 µg/ml of anti-CD248 mAbs (black column) and 0 µg/ml anti-CD248 mAbs (grey column, i.e. w/o mAb). Binding was evaluated by flow cytometry using a secondary anti-human IgG antibody and expressed as Mean Fluorescence Intensity (MFI).

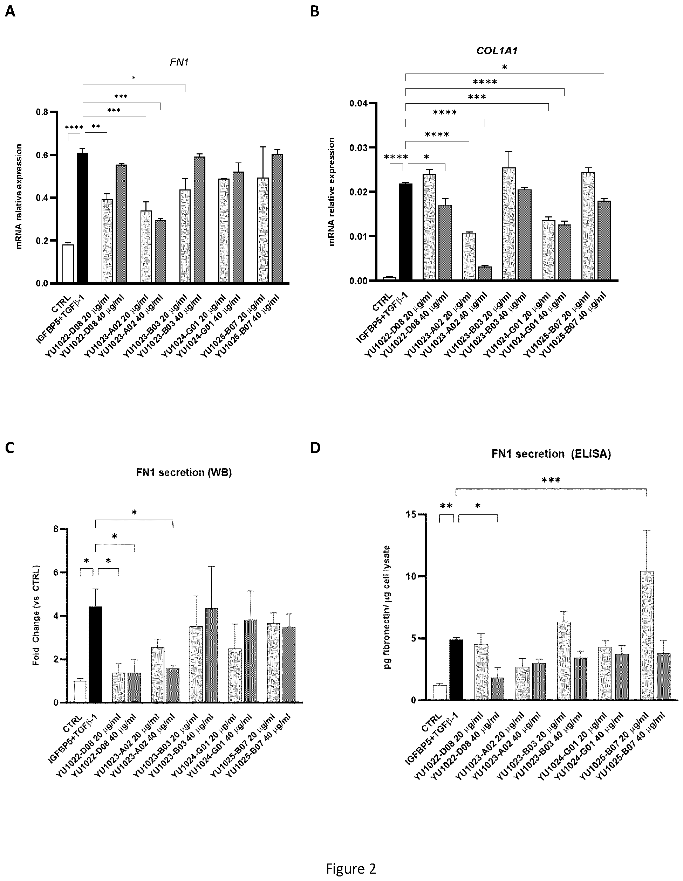

**Figures. 2A-2D** are bar graphs summarizing the effect of anti-CD248 mAbs YU1022-D08, YU1023-A02 YU1023-B03, YU1024-G01 and YU1025-B07 on the modulation of Fibronectin *(FN1)* and collagen1 alpha 1 (*COL1A1*) mRNA expression (panel **A** and **B,** respectively) as well as fibronectin secreted protein (evaluated via Western Blot and ELISA analyses in **C** and **D**) in Alveolar epithelial cells (AECs) following treatment with human recombinant IGFBP5 (500 ng/ml), human recombinant TGF-β1 (5 ng/ml), and/or 20 µg/mL or 40 µg/mL anti-CD248 mAbs.

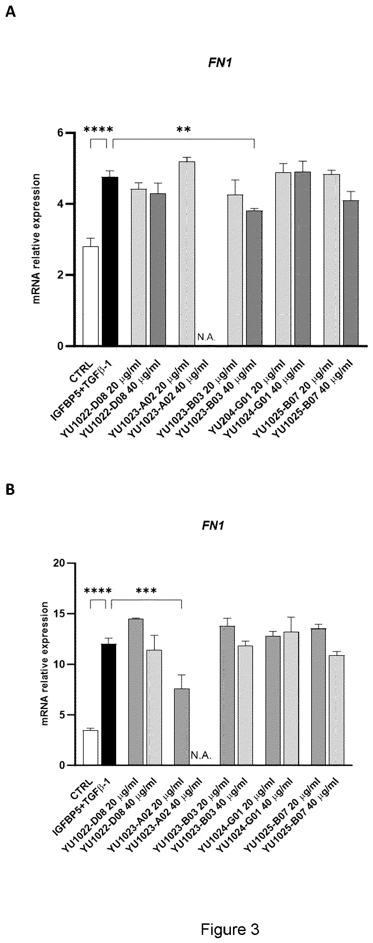

**Figures 3A-3B** are bar graphs summarizing the effect of anti-CD248 mAbs YU1022-D08, YU1023-A02 YU1023-B03, YU1024-G01 and YU1025-B07 on the modulation of Fibronectin *(FN1)* mRNA in Control Primary Human Lung Fibroblasts (CTRL-HLF) and IPF Primary Human Lung Fibroblasts (IPF-HLF) (panel **A** and **B,** respectively) following treatment with human recombinant IGFBP5 (500 ng/ml), human recombinant TGF-β1 (1.25 ng/ml), and/or 20 µg/mL or 40 µg/mL of anti-CD248 mAbs.

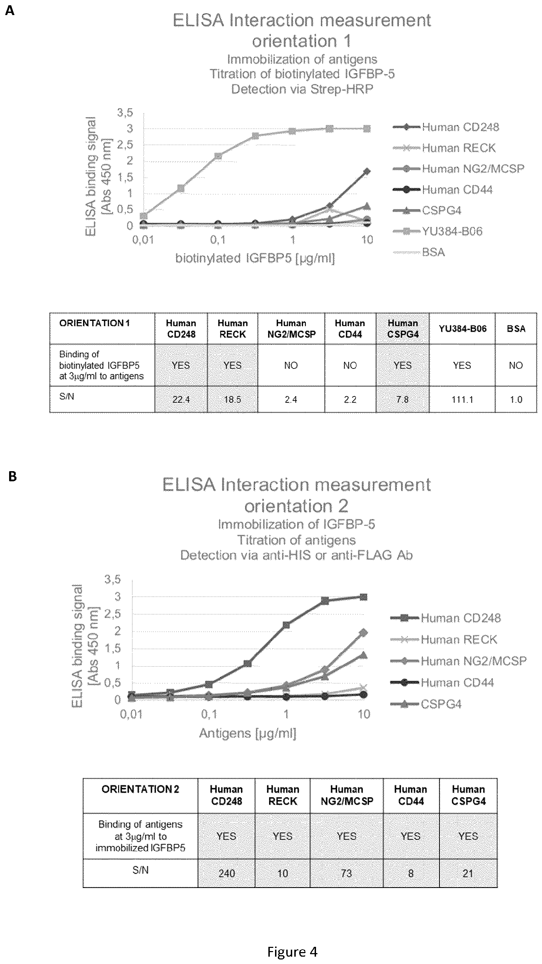

**Figures 4A-4B** are A) ELISA interaction measurement of human IGFBP5 with human CD248, RECK, CSPG4/NG2 and CD44 in orientation 1. In this setting, antigens/receptor were immobilized to the ELISA plate and the binding of biotinylated IGFBP5 to the immobilized antigens/receptor was measured using Streptavidin-HRP. As positive control, a monoclonal antibody specific for IGFBP5 previously developed (YU384-B06) was immobilized on the plate and the binding of biotinylated IGFBP5 to BSA measured using Streptavidin-HRP. As negative control, bovine serum albumin (BSA) was immobilized on the plate and the binding of biotinylated IGFBP5 to BSA measured using Streptavidin-HRP. The signal-to-noise (S/N) ratio was calculated at an IGFBP5 concentration of 3 µg/ml, by dividing the binding signal of IGFBP5+antigen versus IGFBP5+BSA signal. S/N ratios >5 were considered to be associated with significant binding. Data of S/N ratio are summarized in the table in the figure. B) ELISA interaction measurement of human IGFBP5 with human CD248, RECK, CSPG4/NG2 and CD44 in orientation 2. In this setting, IGFBP5 or BSA were immobilized to the ELISA plate and the binding of the different antigens/receptor proteins to the immobilized IGFBP5 or immobilized BSA was measured. The S/N ratio was calculated at an antigen concentration of 3 µg/ml, by dividing the binding signal of IGFBP5+antigen versus BSA+antigen. Data of S/N ratio are summarized in the table below the figure.

### DETAILED DESCRIPTION OF THE INVENTION

Unless otherwise noted, technical terms are used according to conventional usage in the art.

The terms "comprising", "comprises" and "comprised of" as used herein are synonymous with "including" or "includes" or "containing" or "contains", and are inclusive or openended and do not exclude additional, non-recited members, elements or steps. The terms "comprising", "comprises" and "comprised of" also include the term "consisting of". The terms "percent sequence identity" (% sequence identity), "percent identical" (% identical) and the like refer to percent sequence identity between two nucleotide sequences or between two amino acid sequences calculated by aligning the two sequences, determining the number of matches of nucleotides or amino acid residues between the two sequences, dividing the number of matches by the length of the aligned region (i.e., the number of aligned nucleotides or amino acid residues), and multiplying by 100 to arrive at a percent sequence identity value. For calculation of the percent sequence identity (% sequence identity), unless otherwise specified, two or more sequences are aligned using the EMBOSS Needle Pairwise Sequence Alignment software tool based on the Needleman and Wunsch algorithm (available at www.ebi.ac.uk/Tools/psa/emboss_needle) with the following parameters: Matrix: BLOSUM62 (for protein sequences) or DNAfull (for DNA sequences); Gap Open: 10; Gap Extend: 0.5; End Gap Penalty: false; End Gap Open: 10; and End Gap Extend: 0.5. Additional methods of alignment of nucleotide and amino acid sequences for comparison are also well known in the art. The local homology algorithm (BESTFIT) of Smith and Waterman (1981) Adv. Appl. Math 2:482, may permit optimal alignment of compared sequences; by the homology alignment algorithm (GAP) of Needleman and Wunsch (1970) J. Mol. Biol. 48:443-453; by the search for similarity method (Tfasta and Fasta) of Pearson and Lipman (1988) Proc. Natl. Acad. Sci. USA 85:2444; by computerized implementations of these algorithms, including, but not limited to: CLUSTAL in the PC/Gene program by Intelligenetics, Mountain View, Calif., GAP, BESTFIT, BLAST, FASTA and TFASTA in the Wisconsin Genetics Software Package, Version 8 (available from Genetics Computer Group (GCG^{™} programs (Accelrys, Inc., San Diego, Calif.)). The CLUSTAL program is well described by Higgins and Sharp (1988) Gene 73:237-244; Higgins and Sharp (1989) CABIOS 5: 151-153; Corpet, et al. (1988) Nucleic Acids Res. 16: 10881-10890; Huang, et al. (1992) Computer Applications in the Biosciences 8: 155-165; and Pearson, et al. (1994) Meth. Mol. Biol. 24:307-331. An example of a good program to use for optimal global alignment of multiple sequences is PileUp (Feng and Doolittle (1987) J. Mol. Evol. 25:351- 260, which is similar to the method described by Higgins and Sharp (1989) CABIOS 5: 151- 153 (and is hereby incorporated by reference). The BLAST family of programs that can be used for database similarity searches includes: BLASTN for nucleotide query sequences against nucleotide database sequences; BLASTX for nucleotide query sequences against protein database sequences; BLASTP for protein query sequences against protein database sequences; TBLASTN for protein query sequences against nucleotide database sequences; and TBLASTX for nucleotide query sequences against nucleotide database sequences. See, Current Protocols in Molecular Biology, Chapter 19, Ausubel, et al., eds., Greene Publishing and Wiley-Interscience, New York (1995). An updated version of the BLAST family of programs includes the BLAST+ suite. (Camacho, C., et al. (2009 Dec 15) BLAST+: architecture and applications. BMC Bioinformatics 10:421).

In particular, in the present invention "at least 80 % identity" means that the identity may be at least 80%, or 85 %, or 90%, or 95%, or 100% sequence identity to referred sequences wherein the % of sequence identity is calculated as described above. This applies to all the mentioned % of identity. In the present invention "at least 95% identity" means that the identity may be at least 95%, 96%, 97%, 98%, 99%, or 100% sequence identity to referred sequences. This applies to all the mentioned % of identity. In the present invention "at least 98% identity" means that the identity may be at least 98%, 99%, or 100% sequence identity to referred sequences. This applies to all the mentioned % of identity. Preferably, the % of identity relates to the full length of the referred sequence.

The antibodies of the invention specifically bind human CD248.

As discussed herein, these antibodies are collectively referred to as "anti-CD248 antibodies". All of such antibodies are encompassed by the discussion herein. The respective antibodies can be used alone or in combination in the methods of the invention.

By "antibodies that specifically bind" CD248 is intended that the antibodies will not substantially cross react with another, non-homologous, human polypeptide. By "not substantially cross react" is intended that the antibody or fragment has a binding affinity for a non-homologous protein which is less than 10%, more preferably less than 5%, and even more preferably less than 1 %, of the binding affinity for CD248.

In various embodiments, an antibody that "specifically binds" CD248, as used herein, includes antibodies that bind human CD248 with a KD of less than about 1000 nM, less than about 500 nM, less than about 300 nM, less than about 200 nM, less than about 100 nM, less than about 90 nM, less than about 80 nM, less than about 70 nM, less than about 60 nM, less than about 50 nM, less than about 40 nM, less than about 30 nM, less than about 20 nM, less than about 10 nM, less than about 5 nM, less than about 4 nM, less than about 3 nM, less than about 2 nM, less than about 1 nM or about 0.5 nM or about 0.05 nM, as measured with an Octet biolayer interferometry device or in a surface plasmon resonance assay, for example using the BIAcore^{™} system (Biacore Life Sciences division of GE Healthcare, Piscataway, NJ) or kinetic exclusion assays or binding to CD248 receptor expressed by HEK293 cells or any known method in the art. The term "antibody" herein is used in the broadest sense understood in the art, including but not limited to, all polypeptides described as antibodies in Sumit G, Wei W, Tsutomu and Satoshi O, Antibodies 2013; 2: 452-500, incorporated herein by reference and including, but not limited to, all formats described in Martin et al., MAbs 15(1):2191301 (2023).

For example, the term "antibody", as used herein encompasses monoclonal antibodies, polyclonal antibodies, monospecific and multi-specific antibodies (e.g., bispecific antibodies), and antibody fragments so long as the fragment exhibits the desired antigen-binding activity (antigen-binding fragments).

The terms "antigen-binding fragment" of an antibody or equivalently "antigen-binding portion" of an antibody and the like, as used herein, include any naturally occurring, enzymatically obtainable, synthetic, or genetically engineered polypeptide or glycoprotein that comprises a portion of an antibody and that specifically binds an antigen to form a complex. Antigen-binding fragments of an antibody may be derived, e.g., from full antibody molecules using any suitable standard techniques such as proteolytic digestion or recombinant genetic engineering techniques involving the manipulation and expression of DNA encoding antibody variable and optionally constant domains. Such DNA is known and/or is readily available from, e.g., commercial sources, DNA libraries (including, e.g., phage-antibody libraries), or can be synthesized. The DNA may be sequenced and manipulated chemically or by using molecular biology techniques, for example, to arrange one or more variable and/or constant domains into a suitable configuration, or to introduce codons, create cysteine residues, modify, add or delete amino acids, etc.

As with full antibody molecules, antigen-binding fragments may be monospecific or multi-specific (e.g., bispecific). A multi-specific antigen-binding fragment of an antibody will typically comprise at least two different antigen binding moieties, wherein each antigen binding moiety is capable of specifically binding to a separate antigen or to a different epitope on the same antigen.

In particular embodiments, an antigen-binding fragment of an antibody comprises at least one variable domain covalently linked to at least one constant domain. Non-limiting, exemplary configurations of variable and constant domains that may be found within an antigen-binding fragment of an antibody include: (i) VH- CH1; (ii) VH-CH2; (iii) VH-CH3; (iv) VH-CH1-CH2; (v) VH-CH1-CH2-CH3; (vi) VH- CH2-CH3; (vii) VH-CL; (viii) VL-CH1; (ix) VL-CH2; (x) VL-CH3; (xi) VL-CH1-CH2; (xii) VL-CH1-CH2-CH3; (xiii) VL-CH2-CH3; and (xiv) VL-CL. In any configuration of variable and constant domains, including any of the exemplary configurations listed above, the variable and constant domains may be either directly linked to one another or may be linked by a full or partial hinge or linker region. A hinge region may in various embodiments consist of at least 2 (e.g., 5, 10, 15, 20, 40, 60 or more) amino acids which result in a flexible or semi-flexible linkage between adjacent variable and/or constant domains in a single polypeptide molecule. Moreover, an antigen-binding fragment of an antibody may in various embodiments comprise a homo-dimer or hetero-dimer (or other multimer) of any of the variable and constant domain configurations listed above in non-covalent association with one another and/or with one or more monomeric VH or VL domain (e.g., by disulfide bond(s)).

The term "antigen-binding fragment" of an antibody further includes single domain antibodies. A single-domain antibody is an antibody fragment consisting of a single monomeric variable antibody domain. In some embodiments, the single-domain antibody is derived from the variable domain of the antibody heavy chain from camelids (also termed nanobodies, or VHH fragments). In some embodiments, the single-domain antibody is an autonomous human heavy chain variable domain (aVH) or VNAR fragments derived from sharks.

Non-limiting examples of antigen-binding fragments include: (i) Fab fragments; (ii) F(ab')2 fragments; (iii) Fd fragments; (iv) Fv fragments; (v) single-chain Fv (scFv) molecules; (vi) dAb fragments; and (vii) minimal recognition units consisting of the amino acid residues that mimic the hypervariable region of an antibody (e.g., an isolated complementarity determining region (CDR) such as a CDR3 peptide), or a constrained FR3-CDR3-FR4 peptide. Other engineered molecules, such as domain-specific antibodies, single domain antibodies, domain- deleted antibodies, chimeric antibodies, CDR-grafted antibodies, diabodies, triabodies, tetrabodies, minibodies, nanobodies (e.g., monovalent nanobodies, and bivalent nanobodies), small modular immunopharmaceuticals (SMIPs), and shark variable IgNAR domains, are also encompassed within the expression "antigen-binding fragment," as used herein.

An antigen-binding fragment of an antibody will typically comprise at least one variable domain. The variable domain may be of any size or amino acid composition and will generally comprise at least one CDR which is adjacent to or in frame with one or more framework sequences. In antigen-binding fragments having a VH domain associated with a VL domain, the VH and VL domains may be situated relative to one another in any suitable arrangement. For example, the variable region may be dimeric and contain VH-VH, VH-VL or VL-VL dimers. Alternatively, the antigen-binding fragment of an antibody may contain a monomeric VH or VL domain.

The term "antibody," as used herein, also includes ADC (antibody drug conjugate) and payload fusion antibodies.

As used herein, the term "antigen binding molecule" refers in its broadest sense to a molecule that specifically binds an antigenic determinant. Examples of antigen binding molecules are antibodies, including antigen-binding antibody fragments, and scaffold antigen binding proteins.

The term "antigen binding moiety" refers to the portion of an antigen binding molecule that specifically binds to an antigenic determinant. Antigen binding moieties include antibodies and antigen-binding fragments thereof, such as scFv, that are capable of specific binding to an antigen on a target cell. In a particular aspect, the antigen binding moiety is able to direct the entity to which it is attached, such as a cell, to a target site. In addition, antigen binding moieties capable of specific binding to a target cell antigen include scaffold antigen binding proteins as defined herein below, e.g. binding domains which are based on designed repeat proteins or designed repeat domains such as designed ankyrin repeat proteins (DARPins) (see e.g. WO 2002/020565) or Lipocalins (Anticalin). Designed Ankyrin Repeat Proteins (DARPins) are derived from Ankyrin, which is a family of proteins that mediate attachment of integral membrane proteins to the cytoskeleton. A single ankyrin repeat is a 33-residue motif consisting of two alpha-helices and a beta-turn. They can be engineered to bind different target antigens by randomizing residues in the first alpha-helix and a beta-turn of each repeat. Their binding interface can be increased by increasing the number of modules (a method of affinity maturation). For further details see J. Mol. Biol. 332, 489-503 (2003), PNAS 100(4), 1700-1705 (2003) and J. Mol. Biol. 369, 1015-1028 (2007) and US20040132028.

In certain embodiments, antibodies and antigen binding molecules provided herein are altered to increase or decrease the extent to which the antigen binding moiety is glycosylated. Glycosylation variants of the molecules may be conveniently obtained by altering the amino acid sequence such that one or more glycosylation sites is created or removed. Where the antigen binding molecule comprises an Fc region, the carbohydrate attached thereto may be altered. In one aspect, variants of antigen binding molecules are provided having a carbohydrate structure that lacks fucose attached (directly or indirectly) to an Fc region. Such fucosylation variants may have improved ADCC function, see e.g. US Patent Publication Nos. US 2003/0157108 (Presta, L.) or US 2004/0093621 (Kyowa Hakko Kogyo Co., Ltd). Further variants of antigen binding molecules of the invention include those with bisected oligosaccharides, e.g., in which a biantennary oligosaccharide attached to the Fc region is bisected by GlcNAc. Such variants may have reduced fucosylation and/or improved ADCC function, see for example WO 2003/011878 (Jean-Mairet et al.); US Patent No. 6,602,684 (Umana et al.); and US 2005/0123546 (Umana et al.). Variants with at least one galactose residue in the oligosaccharide attached to the Fc region are also provided. Such antibody variants may have improved CDC function and are described, e.g., in WO 1997/30087 (Patel et al.); WO 1998/58964 (Raju, S.) and WO 1999/22764 (Raju, S.).

In certain embodiments, it may be desirable to create cysteine engineered variants of the antibody or antigen binding molecule of the invention, e.g., "thioMAbs," in which one or more residues of the molecule are substituted with cysteine residues. In particular embodiments, the substituted residues occur at accessible sites of the molecule. By substituting those residues with cysteine, reactive thiol groups are thereby positioned at accessible sites of the antibody and may be used to conjugate the antibody to other moieties, such as drug moieties or linker-drug moieties, to create an immunoconjugate. In certain embodiments, any one or more of the following residues may be substituted with cysteine: V205 (Kabat numbering) of the light chain; A118 (EU numbering) of the heavy chain; and S400 (EU numbering) of the heavy chain Fc region. Cysteine engineered antigen binding molecules may be generated as described, e.g., in U.S. Patent No. 7,521,541.

In certain aspects, the antibody or antigen binding molecules provided herein may be further modified to contain additional non-proteinaceous moieties that are known in the art and readily available. The moieties suitable for derivatization of the antibody or antigen binding molecule include but are not limited to water soluble polymers. Non-limiting examples of water soluble polymers include, but are not limited to, polyethylene glycol (PEG), copolymers of ethylene glycol/propylene glycol, carboxymethylcellulose, dextran, polyvinyl alcohol, polyvinyl pyrrolidone, poly-1, 3-dioxolane, poly-l,3,6-trioxane, ethylene/maleic anhydride copolymer, polyaminoacids (either homopolymers or random copolymers), and dextran or poly(n-vinyl pyrrolidone)polyethylene glycol, propropylene glycol homopolymers, prolypropylene oxide/ethylene oxide co-polymers, polyoxyethylated polyols (e.g., glycerol), polyvinyl alcohol, and mixtures thereof. Polyethylene glycol propionaldehyde may have advantages in manufacturing due to its stability in water. The polymer may be of any molecular weight and may be branched or unbranched. The number of polymers attached to the antibody may vary, and if more than one polymer is attached, they can be the same or different molecules. In general, the number and/or type of polymers used for derivatization can be determined based on considerations including, but not limited to, the particular properties or functions of the antibody to be improved, whether the antibody derivative will be used in a therapy under defined conditions, etc.

In another aspect, conjugates of an antibody and non-proteinaceous moiety that may be selectively heated by exposure to radiation are provided. In one embodiment, the non-proteinaceous moiety is a carbon nanotube (Kam, N.W. et al., Proc. Natl. Acad. Sci. USA 102 (2005) 11600-11605). The radiation may be of any wavelength, and includes, but is not limited to, wavelengths that do not harm ordinary cells, but which heat the non-proteinaceous moiety to a temperature at which cells proximal to the antibody-non-proteinaceous moiety are killed. In another aspect, immunoconjugates of the antigen binding molecules provided herein may be obtained. An "immunoconjugate" is an antibody conjugated to one or more heterologous molecule(s), including but not limited to a cytotoxic agent.

The constant region of an antibody is important in the ability of an antibody to fix complement and mediate cell-dependent cytotoxicity. Thus, the isotype of an antibody may be selected on the basis of whether it is desirable for the antibody to mediate cytotoxicity, or not. In certain embodiments, the constant region is an IgG1, IgG2, IgG3, IgG4 constant region.

The instant invention encompasses in various embodiments antibodies having one or more mutations in the hinge, CH2 or CH3 region which may be desirable, for example, in production, to improve the yield of the desired antibody form. In some embodiments, for example, the antibodies described herein comprise a human IgG4 constant region. In particular embodiments, the IgG4 constant region has a single amino acid substitution in the hinge region of the human IgG4 hinge which reduced Fab arm exchange (Angal et al. (1993) Molecular Immunology 30:105) to levels typically observed using a human IgG1 hinge.

In certain embodiments, the antibody comprises one or more mutations in the constant region that increase serum half-life, including those described in US Patent Nos. 7,083,784, 8,323,962 and Dall'Aqua et al., J. Biol. Chem. 281 (33):23514-23524 (2006); Hinton et al., J. Immunology 176:346-356 (2006); Yeung et al., J. Immunology 182:7663-7671 (2009); and Petkova et al., Intn'l Immunology,18: 1759-1769 (2006), incorporated herein by reference in their entireties.

In a preferred embodiment, the antibody molecule includes a heavy chain constant region for an IgG4, e.g., a human IgG4. In a preferred embodiment, the human IgG4 includes a substitution at position 228 (e.g., a Ser to Pro substitution, S228P). In a preferred embodiment, the human IgG4 includes a substitution at position 235 (e.g., a Leu to Glu substitution, L235E). In a preferred embodiment, the human IgG4 includes a substitution at position 228 (e.g., a Ser to Pro substitution) and a substitution at position 235 (e.g., a Leu to Glu substitution). In a preferred embodiment, the antibody includes a S228P substitution and/or a L235E substitution. In still another embodiment, the antibody molecule includes a heavy chain constant region for an IgG1, e.g., a human IgG1. In one embodiment, the human IgG1 includes a substitution at position 297 (e.g., an Asn to Ala substitution). In one embodiment the human IgG1 includes a substitution at position 250, a substitution at position 428, or both (e.g., a Thr to Gln substitution at position 250 and/or a Met to Leu substitution at position 428). In one embodiment, the human IgG1 includes a substitution at position 234, a substitution at position 235, or both (e.g., a Leu to Ala substitution at position 234 and/or a Leu to Ala substitution at position 235). In preferred embodiments, the antibody includes a heavy chain constant region and/or a light chain constant region having one of the amino acid sequences of Table 18.

The term "human antibody", as used herein, is intended to include antibodies having variable and constant regions derived from human germline immunoglobulin sequences. The human antibodies featured in the invention may in various embodiments nonetheless include amino acid residues not encoded by human germline immunoglobulin sequences (e.g., mutations introduced by random or site- specific mutagenesis in vitro or by somatic mutation in vivo), for example in the CDRs and, in some embodiments, CDR3. However, the term "human antibody", as used herein, is not intended to include antibodies in which CDR sequences are derived from the germline of another mammalian species, such as a mouse, which have been grafted onto human framework sequences.

The term "recombinant human antibody", as used herein, is intended to include all human antibodies that are prepared, expressed, created or isolated by recombinant means, such as antibodies expressed using a recombinant expression vector transfected into a host cell (described further below), antibodies isolated from a recombinant, combinatorial human antibody library (described further below), antibodies isolated from an animal (e.g., a mouse) that is transgenic for human immunoglobulin genes or antibodies prepared, expressed, created or isolated by any other means that involves splicing of human immunoglobulin gene sequences to other DNA sequences. Such recombinant human antibodies have variable and constant regions derived from human germline immunoglobulin sequences. In certain embodiments, however, such recombinant human antibodies are subjected to in vitro mutagenesis (or, when an animal transgenic for human Ig sequences is used, in vivo somatic mutagenesis) and thus the amino acid sequences of the VH and VL regions of the recombinant antibodies are sequences that, while derived from and related to human germline VH and VL sequences, may not naturally exist within the human antibody germline repertoire in vivo. An "isolated antibody," as used herein, means an antibody that has been identified and separated and/or recovered from at least one component of its natural environment. For example, an antibody that has been separated or removed from at least one component of an organism, or from a tissue or cell in which the antibody naturally exists or is naturally produced, is an "isolated antibody." In various embodiments, the isolated antibody also includes an antibody in situ within a recombinant cell. In other embodiments, isolated antibodies are antibodies that have been subjected to at least one purification or isolation step. In various embodiments, an isolated antibody may be substantially free of other cellular material and/or chemicals.

The term "epitope" refers to an antigenic determinant that interacts with a specific antigen binding site in the variable region of an antibody molecule known as a paratope. A single antigen may have more than one epitope. Thus, different antibodies may bind to different areas on an antigen and may have different biological effects. Epitopes may be either conformational or linear. A conformational epitope is produced by spatially juxtaposed amino acids from different segments of the linear polypeptide chain. A linear epitope is one produced by adjacent amino acid residues in a polypeptide chain. In certain circumstance, an epitope may include moieties of saccharides, phosphoryl groups, or sulfonyl groups on the antigen.

Still preferably the inhibitor as defined above i) reduces or inhibits extracellular matrix production and/or ii) reduces or inhibits extracellular matrix deposition and/or iii) reduces or inhibits collagen and/or fibronectin production in primary lung fibroblasts and/or lung epithelial cells and/or iv) reduces or inhibits collagen and/or fibronectin deposition in primary lung fibroblasts and/or lung epithelial cells.

In an embodiment, the antibody molecule includes a heavy chain variable domain and a constant region, a light chain variable domain and a constant region, or both, comprising the amino acid sequence of any of YU1022-D08, YU1023-A02, YU1023-B03, YU1023-E03, YU1024-G01 and YU1025-B07 as defined in Tables 4-15, 18 or encoded by a nucleotide sequence in Tables 16 or 17; or a sequence substantially identical (e.g., at least 80%, 85%, 90%, 92%, 95%, 97%, 98%, 99% or higher identical) to any of the aforesaid sequences. The antibody molecule, optionally, comprises a leader sequence from a heavy chain, a light chain, or both.

In yet another embodiment, the antibody molecule includes at least one, two, or three complementarity determining regions (CDRs) from a heavy chain variable region of an antibody described herein, e.g., an antibody chosen from any of any of YU1022-D08, YU1023-A02, YU1023-B03, YU1023-E03, YU1024-G01 and YU1025-B07 as defined in Tables 4-5 or 8-15 or encoded by the nucleotide sequence in Tables 16-17; or a sequence substantially identical (e.g., at least 80%, 85%, 90%, 92%, 95%, 97%, 98%, 99% or higher identical) to any of the aforesaid sequences.

In yet another embodiment, the antibody molecule includes at least one, two, or three CDRs (or collectively all of the CDRs) from a heavy chain variable region comprising an amino acid sequence shown in Tables 4, 6, 8-13 or encoded by a nucleotide sequence shown in Table 16. In one embodiment, one or more of the CDRs (or collectively all of the CDRs) have one, two, three, four, five, six or more changes, e.g., amino acid substitutions or deletions, relative to the amino acid sequence shown in Tables 4, 8-13 or encoded by a nucleotide sequence shown in Table 15. In certain embodiments, the antibody molecule includes a substitution in a heavy chain CDR, e.g., one or more substitutions in a CDR1, CDR2 and/or CDR3 of the heavy chain.

In yet another embodiment, the antibody molecule includes at least one, two, or three CDRs from a light chain variable region of an antibody described herein, e.g., an antibody chosen from any of any of as defined in Tables 5, 8-13 or encoded by the nucleotide sequence in Table 17; or a sequence substantially identical (e.g., at least 80%, 85%, 90%, 92%, 95%, 97%, 98%, 99% or higher identical) to any of the aforesaid sequence. In certain embodiments, the antibody molecule includes a substitution in a light chain CDR, e.g., one or more substitutions in a CDR1, CDR2 and/or CDR3 of the light chain.

In another embodiment, the antibody molecule includes at least one, two, three, four, five or six CDRs (or collectively all of the CDRs) from a heavy and light chain variable region comprising an amino acid sequence shown in Tables 4-13, or encoded by a nucleotide sequence shown in Tables 16-17. In one embodiment, one or more of the CDRs (or collectively all of the CDRs) have one, two, three, four, five, six or more changes, e.g., amino acid substitutions or deletions, relative to the amino acid sequence shown in Tables 4-13, or encoded by a nucleotide sequence shown in Tables 16-17.

In one embodiment, the antibody molecule includes all six CDRs from an antibody described herein, e.g., an antibody chosen from any of any of YU1022-D08, YU1023-A02, YU1023-B03, YU1023-E03, YU1024-G01 and YU1025-B07 as defined in Tables 4-15 or encoded by the nucleotide sequence in Tables 16-17 or closely related CDRs, e.g., CDRs which are identical or which have at least one amino acid alteration, but not more than two, three or four alterations (e.g., substitutions, deletions, or insertions, e.g., conservative substitutions). In one embodiment, the antibody molecule may include any CDR described herein.

In one embodiment, one or more of the CDRs (or collectively all of the CDRs) have one, two, three, four, five, six or more changes, e.g., amino acid substitutions or deletions, relative to the amino acid sequence shown in Tables 4-13, in particular in Tables 4-5. In certain embodiments, the antibody includes a substitution in a light chain CDR, e.g., one or more substitutions in a CDR1, CDR2 and/or CDR3 of the light chain.

In certain embodiments, the antibody includes a substitution in a heavy chain CDR, e.g., one or more substitutions in a CDR1, CDR2 and/or CDR3 of the heavy chain.

In certain embodiments, the antibody includes from 1 to 5 amino acid substitutions among the six CDRs, i.e. CDR1, CDR2, CDR3 of the heavy chain, CDR1, CDR2 and CDR3 of the light chain.

Preferably, the term "CDR" is a CDR as defined by Kabat, based on sequence comparisons. CDRH1, CDRH2 and CDRH3 denote the heavy chain CDRs, and CDRL1, CDRL2 and CDRL3 denote the light chain CDRs.

In an embodiment, the antibody molecule includes at least one, two, or three CDRs according to Kabat et al., e.g., at least one, two, or three CDRs according to the Kabat definition as set out in any one of Tables 8-13, from a heavy chain variable region of an antibody described herein, or a sequence substantially identical (e.g., at least 80%, 85%, 90%, 92%, 95%, 97%, 98%, 99% or higher identical) to any of the aforesaid sequences; or which have at least one amino acid alteration, but not more than two, three or four alterations (e.g., substitutions, deletions, or insertions, e.g., conservative substitutions) relative to one, two, or three CDRs according to Kabat et al. shown in Tables 8-13.

In another embodiment, the antibody molecule includes at least one, two, or three CDRs according to Kabat et al., e.g., at least one, two, or three CDRs according to the Kabat definition as set out in any one of Tables 8-13 from a light chain variable region of an antibody described herein, or a sequence substantially identical (e.g., at least 80%, 85%, 90%, 92%, 95%, 97%, 98%, 99% or higher identical) to any of the aforesaid sequences; or which have at least one amino acid alteration, but not more than two, three or four alterations (e.g., substitutions, deletions, or insertions, e.g., conservative substitutions) relative to one, two, or three CDRs according to Kabat et al. shown in Tables 8-13.

In yet another embodiment, the antibody molecule includes all six CDRs according to Kabat et al., e.g., all six CDRs according to the Kabat definition as set out in Tables 8-13, from the heavy and light chain variable regions of an antibody described herein, e.g., an antibody chosen from any of YU1022-D08, YU1023-A02, YU1023-B03, YU1023-E03, YU1024-G01 and YU1025-B07 as defined in Tables 4-13 or encoded by the nucleotide sequence in Tables 16-17; or a sequence substantially identical (e.g., at least 80%, 85%, 90%, 92%, 95%, 97%, 98%, 99% or higher identical) to any of the aforesaid sequences; or which have at least one amino acid alteration, but not more than two, three or four alterations (e.g., substitutions, deletions, or insertions, e.g., conservative substitutions) relative to all six CDRs according to Kabat definition. In one embodiment, the antibody molecule may include any CDR described herein.

In another embodiment, the antibody includes at least one, two, or three CDRs of a heavy chain variable region according to Chothia, AbM, Contact or IMGT definition as disclosed in Tables 8-13; or which have at least one amino acid alteration, but not more than two, three or four alterations (e.g., substitutions, deletions, or insertions, e.g., conservative substitutions).

In another embodiment, the antibody includes at least one, two, or three CDRs of a light chain variable region according to Chothia, AbM, Contact or IMGT definition as disclosed in Tables 8-13; or which have at least one amino acid alteration, but not more than two, three or four alterations (e.g., substitutions, deletions, or insertions, e.g., conservative substitutions).

In certain embodiments, the antibody molecule includes a combination of CDRs defined according to the Kabat, Chothia, AbM, Contact or IMGT definition.

Preferred antibodies are antibodies YU1022-D08, YU1023-A02, YU1023-B03, YU1023-E03, YU1024-G01 and YU1025-B07 as defined in Tables 4-15, 18 or encoded by the nucleotide sequence in Tables 16 and 17.

In one embodiment, the antibody includes:
(i) a heavy chain variable region (VH) including a VHCDR1 amino acid sequence chosen from any one of SEQ ID NO: 1, 2 and 3; a VHCDR2 amino acid sequence chosen from any one of SEQ ID NO: 4, 5 and 6; and a VHCDR3 amino acid sequence chosen from any one of SEQ ID NO: 7, 8, 9, 10, 11 and 12; and
(ii) a light chain variable region (VL) including a VLCDR1 amino acid sequence chosen from any one of SEQ ID NO: 13, 14, 15, 16, 17 and 18, a VLCDR2 amino acid sequence chosen from any one of SEQ ID NO: 19, 20, 21, 22 and 23, and a VLCDR3 amino acid sequence chosen from SEQ ID NO: 24, 25, 26, 27, 28 and 29.

In one embodiment, the light or the heavy chain variable framework (e.g., the region encompassing at least FR1, FR2, FR3, and optionally FR4) of the antibody molecule can be chosen from: (a) a light or heavy chain variable framework including at least 80%, 85%, 87% 90%, 92%, 93%, 95%, 97%, 98%, or preferably 100% of the amino acid residues from a human light or heavy chain variable framework, e.g., a light or heavy chain variable framework residue from a human mature antibody, a human germline sequence, or a human consensus sequence; (b) a light or heavy chain variable framework including from 20% to 80%, 40% to 60%, 60% to 90%, or 70% to 95% of the amino acid residues from a human light or heavy chain variable framework, e.g., a light or heavy chain variable framework residue from a human mature antibody, a human germline sequence, or a human consensus sequence; (c) a non-human framework (e.g., a rodent framework); or (d) a non-human framework that has been modified, e.g., to remove antigenic or cytotoxic determinants, e.g., deimmunized, or partially humanized. In one embodiment, the light or heavy chain variable framework region (particularly FR1, FR2 and/or FR3) includes a light or heavy chain variable framework sequence at least 70, 75, 80, 85, 87, 88, 90, 92, 94, 95, 96, 97, 98, 99% identical or identical to the frameworks of a VL or VH segment of a human germline gene.

In an embodiment, the antibody molecule includes one or more heavy chain framework region (e.g., any of VHFW1 (type a), VHFW1 (type b), VHFW1 (type c), VHFW1 (type d), VHFW2 (type a), VHFW2 (type a'), VHFW2 (type b), VHFW2 (type c), VHFW2 (type d), VHFW2 (type e), VHFW3 (type a), VHFW3 (type b), VHFW3 (type c), VHFW3 (type d), VHFW3 (type e), or VHFW4, or any combination thereof, e.g., a framework combination as described herein for any of YU1022-D08, YU1023-A02, YU1023-B03, YU1023-E03, YU1024-G01 and YU1025-B07 as defined in Table 14, or a sequence substantially identical thereto (e.g., a sequence at least about 85%, 90%, 95%, 99% or more identical thereto, or which differs by no more than 3, 6, 15, 30, or 45 amino acids from the sequences shown in Table 14).

In another embodiment, the antibody molecule includes one or more light chain framework region (e.g., any of VLFW1 (type a), VLFW1 (type b), VLFW1 (type c), VLFW1 (type d), VLFW1 (type e), VLFW1 (type f), VLFW2 (type a), VLFW2 (type c), VLFW3 (type a), VLFW3 (type b), VLFW3 (type c), VLFW3 (type d), VLFW3 (type e), VLFW3 (type f), VLFW3 (type g), or VLFW4, or any combination thereof, e.g., a framework combination as described herein for of any of YU1022-D08, YU1023-A02, YU1023-B03, YU1023-E03, YU1024-G01 and YU1025-B07 as defined in Table 15, or a sequence substantially identical thereto (e.g., a sequence at least about 85%, 90%, 95%, 99% or more identical thereto, or which differs by no more than 3, 6, 15, 30, or 45 amino acids from the sequences shown in Table 15).

In another embodiment, the antibody molecule includes one or more heavy chain framework regions and one or more light chain framework regions as described herein. In certain embodiments, the antibody molecule comprises a heavy chain variable domain having at least one, two, three, four, five, six, seven, ten, fifteen, twenty or more changes, e.g., amino acid substitutions or deletions with respect to the sequence of : SEQ ID NO: 30, SEQ ID NO: 31, SEQ ID NO: 32, SEQ ID NO: 33, SEQ ID NO: 34 or SEQ ID NO: 35.

In certain embodiments, the antibody molecule comprises a light chain variable domain having at least one, two, three, four, five, six, seven, ten, fifteen, twenty or more changes, e.g., amino acid substitutions or deletions with respect to the sequence of SEQ ID NO: 36, SEQ ID NO: 37, SEQ ID NO: 38, SEQ ID NO: 39, SEQ ID NO: 40 or SEQ ID NO: 41.

In one embodiment, the heavy or light chain variable region, or both, of the antibody molecule includes an amino acid sequence encoded by a nucleic acid sequence described herein or a nucleic acid that hybridizes to a nucleic acid sequence described herein, in particular a nucleic acid sequence as shown in Tables 16 and 17 or its complement, e.g., under low stringency, medium stringency, or high stringency, or other hybridization condition described herein.

In another embodiment, the antibody molecule comprises at least one, two, three, or four antigen-binding regions, e.g., variable regions, having an amino acid sequence as set forth in anyone of Tables 4-15 or a sequence substantially identical thereto (e.g., a sequence at least about 85%, 90%, 95%, 99% or more identical thereto, or which differs by no more than 1, 2, 5, 10, or 15 amino acid residues from the sequences shown in Tables 4-15).

In another embodiment, the antibody molecule includes a VH and/or VL domain encoded by a nucleic acid having a nucleotide sequence as set forth in Tables 16 and 17 or a sequence substantially identical thereto (e.g., a sequence at least about 85%, 90%, 95%, 99% or more identical thereto, or which differs by no more than 3, 6, 15, 30, or 45 nucleotides from the sequences shown in Tables 16-17).

In yet other embodiments, the antibody molecule has a heavy chain constant region (Fc) chosen from, e.g., the heavy chain constant regions of IgG1, IgG2, IgG3, IgG4, IgM, IgA1, IgA2, IgD, and IgE; particularly, chosen from, e.g., the heavy chain constant regions of IgG1, IgG2, IgG3, and IgG4, more particularly, the heavy chain constant region of IgG1 or IgG4 (e.g., human IgG1, IgG2 or IgG4). In one embodiment, the heavy chain constant region is human IgG1. In another embodiment, the antibody molecule has a light chain constant region chosen from, e.g., the light chain constant regions of kappa or lambda. In one embodiment, the constant region is altered, e.g., mutated, to modify the properties of the antibody (e.g., to increase or decrease one or more of: Fc receptor binding, antibody glycosylation, the number of cysteine residues, effector cell function, complement function, half-life, aggregation, and stability). In certain embodiments, the antibody comprises a human IgG4 mutated. In a preferred embodiment, the antibody has a heavy chain constant region comprising or consisting of a sequence with SEQ ID NO. 54, SEQ ID NO. 55 or SEQ ID NO. 62. In a preferred embodiment, the antibody has a light chain constant region comprising or consisting of a sequence with SEQ ID NO. 56, SEQ ID NO. 57 or SEQ ID NO. 58.

In one embodiment, the antibody is isolated or recombinant.

In one embodiment, the antibody is a humanized or human antibody molecule.

The antibody of the invention may have framework sequences from any species. Preferably, it may have a mouse or human framework. As used herein the term "framework (FR) amino acid residues" refers to those amino acids in the framework region of an immunoglobulin chain. The term "framework region" or "FR region" as used herein, includes the amino acid residues that are part of the variable region, but are not part of the CDRs (e.g., using the Kabat definition of CDRs).

A monoclonal antibody comprising the CDRs sequences of any of the antibody herein described is also within the scope of the invention.

Methods for producing a monoclonal antibody with the CDR sequences as mentioned above are known in the art and include the introduction of the nucleic acid sequences encoding the CDRs into suitable expression vectors encoding the desired framework sequences.

The anti-CD248 antibodies described herein and useful for the methods featured herein may in various embodiments include one or more amino acid substitutions, insertions and/or deletions in the framework and/or CDR regions of the heavy and light chain variable domains as compared to the corresponding germline sequences from which the antibodies were derived. Such mutations can be readily ascertained by comparing the amino acid sequences disclosed herein to germline sequences available from, for example, public antibody sequence databases.

The present invention includes in various embodiments antibodies and methods involving the use of antibodies, and antigen-binding fragments thereof, which are derived from any of the amino acid sequences disclosed herein, wherein one or more amino acids within one or more framework and/or CDR regions are mutated to the corresponding residue(s) of the germline sequence from which the antibody was derived, or to the corresponding residue(s) of another human germline sequence, or to a conservative amino acid substitution of the corresponding germline residue(s) (such sequence changes are referred to herein collectively as "germline mutations"). Numerous antibodies and antigen- binding fragments may be constructed which comprise one or more individual germline mutations or combinations thereof. In certain embodiments, all of the framework and/or CDR residues within the VH and/or VL domains are mutated back to the residues found in the original germline sequence from which the antibody was derived. In other embodiments, only certain residues are mutated back to the original germline sequence, e.g., only the mutated residues found within the first 8 amino acids of FR1 or within the last 8 amino acids of FR4, or only the mutated residues found within CDR1, CDR2 or CDR3. In other embodiments, one or more of the framework and/or CDR residue(s) are mutated to the corresponding residue(s) of a different germline sequence (i.e., a germline sequence that is different from the germline sequence from which the antibody was originally derived). Furthermore, the antibodies may contain any combination of two or more germline mutations within the framework and/or CDR regions, e.g., wherein certain individual residues are mutated to the corresponding residue of a certain germline sequence while certain other residues that differ from the original germline sequence are maintained or are mutated to the corresponding residue of a different germline sequence. Once obtained, antibodies and antigen-binding fragments that contain one or more germline mutations can be easily tested for one or more desired property such as, improved binding specificity, increased binding affinity, improved or enhanced antagonistic or agonistic biological properties (as the case may be), reduced immunogenicity, etc. The use of antibodies and antigen-binding fragments obtained in this general manner are encompassed within the present invention.

The antibody according to the invention may form a homo- or heterodimer or a homo- or heteromultimer, whereby "dimer" and "multimer" means that two and at least three antibodies, respectively, may combine to form a complex. The prefix "homo" means that a complex may be formed of identical antibody molecules, whereby the prefix "hetero" means that a complex may be formed of different antibody molecules. In general, the term "antibody" is intended to comprise all above-mentioned immunoglobulin isotypes, i.e. the antibody may be an IgA, IgD, IgE, IgG or IgM antibody, including any subclass of these isotypes. Preferably, the antibody is an IgG antibody, more preferred the antibody is an IgG1 antibody. Since the antibody may be expressed and produced recombinantly, the antibody may also comprise two different constant regions of heavy chains, e.g. one IgG1 and one IgG2 heavy chain, or heavy chains from different species. However, the heavy chains preferably are from the same species.

To generate whole antibodies, PCR primers including VH or VL nucleotide sequences, a restriction site, and a flanking sequence to protect the restriction site can be used to amplify the VH or VL sequences in scFv clones or other clones. Utilizing cloning techniques known to those of skill in the art, the PCR amplified VH domains can be cloned into vectors expressing a heavy chain constant region, e.g., the human gamma 4 constant region, and the PCR amplified VL domains can be cloned into vectors expressing a light chain constant region, e.g., human kappa or lambda constant regions. In certain embodiments, the vectors for expressing the VH or VL domains comprise a promoter, a secretion signal, a cloning site for the variable region, constant domains, and a selection marker such as neomycin. The VH and VL domains can also be cloned into one vector expressing the necessary constant regions. The heavy chain conversion vectors and light chain conversion vectors are then co- transfected into cell lines to generate stable or transient cell lines that express full-length antibodies, e.g., IgG, using techniques known to those of skill in the art.

The invention also features a nucleic acid molecule that comprises one or both nucleotide sequences that encode heavy and light chain variable regions, CDRs, framework regions of the antibody, as described herein. In certain embodiments, the nucleotide sequence that encodes the antibody molecule is codon optimized. For example, the invention features a first and second nucleic acid encoding heavy and light chain variable regions, respectively, of an antibody molecule chosen from one or more of, e.g., any of YU1022-D08, YU1023-A02, YU1023-B03, YU1023-E03, YU1024-G01 and YU1025-B07 as defined in Tables 4-15 or encoded by the nucleotide sequence in Tables 16 and 17, or a sequence substantially identical thereto. For example, the nucleic acid can comprise a nucleotide sequence as set forth in Tables 16 and 17, or a sequence substantially identical thereto (e.g., a sequence at least about 85%, 90%, 95%, 99% or more identical thereto, or which differs by no more than 3, 6, 15, 30, or 45 nucleotides from the sequences shown in Tables 16 and 17).

In other embodiments, the nucleic acid molecule comprises a nucleotide sequence that encodes a heavy chain variable domain and/or a heavy chain constant region comprising the amino acid sequence of the heavy chain of any of antibody YU1022-D08, YU1023-A02, YU1023-B03, YU1023-E03, YU1024-G01 or YU1025-B07 as defined in Table 6 or 18 or a nucleotide sequence as defined in Table 16; or a sequence substantially identical (e.g., a sequence at least about 85%, 90%, 95%, 99% or more identical) to any of the aforesaid sequences.

In other embodiments, the nucleic acid molecule comprises a nucleotide sequence that encodes a light chain variable domain and/or a light chain constant region comprising the amino acid sequence of the light chain of any of antibody YU1022-D08, YU1023-A02, YU1023-B03, YU1023-E03, YU1024-G01 or YU1025-B07 as defined in Table 7 or 18 or a nucleotide sequence as defined in Table 17, or a sequence substantially identical (e.g., a sequence at least about 85%, 90%, 95%, 99% or more identical) to any of the aforesaid sequences.

Preferably, said nucleic acid molecule comprises a nucleotide sequence selected from the group consisting of: SEQ ID NO: 42, SEQ ID NO: 43, SEQ ID NO: 44, SEQ ID NO: 45, SEQ ID NO: 46 and SEQ ID NO:47 encoding for a heavy chain variable domain and/or a nucleotide sequence selected from the group consisting of: SEQ ID NO:48, SEQ ID NO: 49, SEQ ID NO: 50, SEQ ID NO: 51, SEQ ID NO: 52 and SEQ ID NO:53 encoding for a light chain variable domain.

The aforesaid nucleotide sequences encoding the herein disclosed heavy and light chain variable domain and constant regions can be present in separate nucleic acid molecules or in the same nucleic acid molecule. In certain embodiments, the nucleic acid molecules comprise a nucleotide sequence encoding a leader sequence.

In certain embodiments, the nucleic acid molecule comprises a nucleotide sequence encoding at least one, two, or three CDRs from a heavy chain variable region having an amino acid sequence as set forth in Tables 4, 8-13, or a sequence substantially homologous thereto (e.g., a sequence at least about 85%, 90%, 95%, 99% or more identical thereto, and/or having one, two, three or more substitutions, insertions or deletions, e.g., conserved substitutions).

In another embodiment, the nucleic acid molecule comprises a nucleotide sequence encoding at least one, two, or three CDRs, or hypervariable loops, from a light chain variable region having an amino acid sequence as set forth in Tables 5, 8-13 or a sequence substantially homologous thereto (e.g., a sequence at least about 85%, 90%, 95%, 99% or more identical thereto, and/or having one, two, three or more substitutions, insertions or deletions, e.g., conserved substitutions).

In yet another embodiment, the nucleic acid molecule comprises a nucleotide sequence encoding at least one, two, three, four, five, or six CDRs from heavy and light chain variable regions having an amino acid sequence as set forth in Tables 4-5, 8-13 or a sequence substantially homologous thereto (e.g., a sequence at least about 85%, 90%, 95%, 99% or more identical thereto, and/or having one, two, three or more substitutions, insertions or deletions, e.g., conserved substitutions).

In another embodiment, the nucleic acid molecule includes one or more heavy chain framework region (e.g., any of VHFW1 (type a), VHFW1 (type b), VHFW1 (type c), VHFW1 (type d), VHFW2 (type a), VHFW2 (type a'), VHFW2 (type b), VHFW2 (type c), VHFW2 (type d), VHFW2 (type e), VHFW3 (type a), VHFW3 (type b), VHFW3 (type c), VHFW3 (type d), VHFW3 (type e), or VHFW4, or any combination thereof, e.g., a framework combination as described herein for any of YU1022-D08, YU1023-A02, YU1023-B03, YU1023-E03, YU1024-G01 and YU1025-B07 as defined in Table 14, or a sequence substantially identical thereto. For example, the nucleic acid molecule can comprise a nucleotide sequence as set forth in Table 16, or a sequence substantially identical thereto (e.g., a sequence at least about 85%, 90%, 95%, 99% or more identical thereto, or which differs by no more than 3, 6, 15, 30, or 45 nucleotides from the sequences shown in Table 16).

In another embodiment, the nucleic acid molecule includes one or more light chain framework region (e.g., any of VLFW1 (type a), VLFW1 (type b), VLFW1 (type c), VLFW1 (type d), VLFW1 (type e), VLFW1 (type f), VLFW2 (type a), VLFW2 (type c), VLFW3 (type a), VLFW3 (type b), VLFW3 (type c), VLFW3 (type d), VLFW3 (type e), VLFW3 (type f), VLFW3 (type g), or VLFW4, or any combination thereof, e.g., a framework combination as described herein for of any of YU1022-D08, YU1023-A02, YU1023-B03, YU1023-E03, YU1024-G01 and YU1025-B07 as defined in Table 15, or a sequence substantially identical thereto. For example, the nucleic acid molecule can comprise a nucleotide sequence as set forth in Table 17, or a sequence substantially identical thereto (e.g., a sequence at least about 85%, 90%, 95%, 99% or more identical thereto, or which differs by no more than 3, 6, 15, 30, or 45 nucleotides from the sequences shown in Table 17).

In another embodiment, the nucleic acid molecule includes one or more heavy chain framework regions and one or more light chain framework regions as described herein.

The heavy and light chain framework regions may be present in the same vector or separate vectors.

In another aspect, the application features host cells and vectors containing the nucleic acids described herein or modified for codon optimization according to known methods. The nucleic acids may be present in a single vector or separate vectors present in the same host cell or separate host cell. The host cell can be a eukaryotic cell, e.g., a mammalian cell, an insect cell, a yeast cell, or a prokaryotic cell, e.g., E. coli. For example, the mammalian cell can be a cultured cell or a cell line. Exemplary mammalian cells include lymphocytic cell lines (e.g., NSO), Chinese hamster ovary cells (CHO), COS cells, HEK 293 cells, oocyte cells, and cells from a transgenic animal, e.g., mammary epithelial cell.

Generally, expression vectors are plasmids which are used to introduce a desired nucleic acid sequence, such as a gene, into a target cell, resulting in the transcription and translation of the protein encoded by the nucleic acid sequence, i.e. the chimeric antigen receptor, the antibody or the binding molecule. Therefore, the expression vector in general comprises regulatory sequences, such as promoter and enhancer regions, as well as a polyadenylation site in order to direct efficient transcription of the nucleic acid sequence on the expression vector. The expression vector may further comprise additional necessary or useful regions, such as a selectable marker for selection in eukaryotic or prokaryotic cells, a purification tag for the purification of the resulting protein, a multiple cloning site or an origin of replication.

Usually, the expression vector may be a viral or a non- viral vector. In general, various kinds of viral vectors, such as retroviral vectors, e.g. lentiviral or adenoviral vectors, or plasmids may be used. In a preferred embodiment, the expression vector according to aspect five is a viral vector. In a more preferred embodiment, the expression vector is a lentiviral vector.

In one aspect, the invention features a method of providing an antibody molecule described herein. The method includes: providing a CD248 antigen (e.g., an antigen comprising at least a portion of a CD248 epitope); obtaining an antibody molecule that specifically binds to the CD248 polypeptide; and evaluating if the antibody molecule specifically binds to the CD248 polypeptide, or evaluating efficacy of the antibody molecule in modulating, e.g., inhibiting, the activity of the CD248. The method can further include administering the antibody molecule to a subject, e.g., a human or non-human animal.

### Therapeutic Methods and Pharmaceutical Compositions

In another aspect, the invention provides compositions, e.g., pharmaceutical compositions, which include a pharmaceutically acceptable carrier, excipient or stabilizer, and at least one of the antibody molecules described herein or antigen-binding fragment thereof. In one embodiment, the composition, e.g., the pharmaceutical composition, includes a combination of the antibody molecule or antigen-binding fragment and one or more agents, e.g., a second therapeutic agent or other antibody molecule, as described herein. In one embodiment, the antibody molecule is conjugated to a label or a therapeutic agent.

The term "bioequivalent" as used herein, refers to a molecule having similar bioavailability (rate and extent of availability) after administration at the same molar dose and under similar conditions (e.g., same route of administration), such that the effect, with respect to both efficacy and safety, can be expected to be essentially same as the comparator molecule. Two pharmaceutical compositions comprising an anti-CD248 antibody are bioequivalent if they are pharmaceutically equivalent, meaning they contain the same amount of active ingredient (e.g., anti-CD248 antibody), in the same dosage form, for the same route of administration and meeting the same or comparable standards. Bioequivalence can be determined, for example, by an in vivo study comparing a pharmacokinetic parameter for the two compositions. Parameters commonly used in bioequivalence studies include peak plasma concentration (Cmax) and area under the plasma drug concentration time curve (AUC).

The antibody or antigen-binding fragment is administered to the subject in various embodiments in a formulation comprising suitable carriers, excipients, and other agents to provide improved transfer, delivery, tolerance, and the like, and suitable for an intravenous or subcutaneous injection.

The injectable preparations may be prepared by methods publicly known. For example, injectable preparations may be prepared, e.g., by dissolving, suspending or emulsifying the antibody or its salt described above in a sterile aqueous medium or an oily medium conventionally used for injections. As the aqueous medium for injections, there are, for example, physiological saline, an isotonic solution containing glucose and other auxiliary agents, etc., which may be used in combination with an appropriate solubilizing agent such as an alcohol (e.g., ethanol), a polyalcohol (e.g., propylene glycol, polyethylene glycol), a nonionic surfactant [e.g., polysorbate 20 or 80, HCO-50 (polyoxyethylene (50 mol) adduct of hydrogenated castor oil)], etc. As the oily medium, there are employed, e.g., sesame oil, soybean oil, etc., which may be used in combination with a solubilizing agent such as benzyl benzoate, benzyl alcohol, etc. The injectable preparation thus prepared can be filled in an appropriate ampoule.

The antibody or antigen-binding fragment according to the invention can be administered to the subject using any acceptable device or mechanism. For example, the administration can be accomplished using a syringe and needle or with a reusable pen and/or autoinjector delivery device. The methods of the present invention include the use of numerous reusable pen and/or autoinjector delivery devices to administer an antibody (or pharmaceutical formulation comprising the antibody). Examples of such devices include, but are not limited to AUTOPEN^{™} (Owen Mumford, Inc., Woodstock, UK), DISETRONIC^{™} pen (Disetronic Medical Systems, Bergdorf, Switzerland), HUMALOG MIX 75/25^{™} pen, HUMALOG^{™} pen, HUMALIN 70/30^{™} pen (Eli Lilly and Co., Indianapolis, IN), NOVOPEN^{™} I, II and III (Novo Nordisk, Copenhagen, Denmark), NOVOPEN JUNIOR^{™} (Novo Nordisk, Copenhagen, Denmark), BD^{™} pen (Becton Dickinson, Franklin Lakes, NJ), OPTIPEN^{™}, OPTIPEN PRO^{™}, OPTIPEN STARLET^{™}, and OPTICLIK^{™} (Sanofi-Aventis, Frankfurt, Germany), to name only a few. Examples of disposable pen and/or autoinjector delivery devices having applications in subcutaneous delivery of a pharmaceutical composition of the present invention include, but are not limited to, the SOLOSTAR^{™} pen (Sanofi-Aventis), the FLEXPEN^{™} (Novo Nordisk), and the KWIKPEN^{™} (Eli Lilly), the SURECLICK^{™} Autoinjector (Amgen, Thousand Oaks, CA), the PENLET^{™} (Haselmeier, Stuttgart, Germany), the EPIPEN (Dey, L.P.), the HUMIRA^{™} Pen (Abbott Labs, Abbott Park, IL), the DAI^{®} Auto Injector (SHL Group) and any auto-injector featuring the PUSHCLICK^{™} technology (SHL Group), to name only a few.

In one embodiment, the antibody or antigen-binding fragment is administered with a prefilled syringe. In another embodiment, the antibody is administered with a prefilled syringe containing a safety system. For example, the safety system prevents an accidental needlestick injury. In various embodiments, the antibody is administered with a prefilled syringe containing an ÈRIS^{™} safety system (West Pharmaceutical Services Inc.). See also U.S. patent numbers 5,215,534 and 9,248,242, incorporated herein by reference in their entireties.

In another embodiment, the antibody or antigen-binding fragment is administered with an auto-injector. In various embodiments, the antibody is administered with an auto-injector featuring the PUSHCLICK^{™} technology (SHL Group). In various embodiments, the auto-injector is a device comprising a syringe that allows for administration of a dose of the composition and/or antibody to a subject. See also U.S. patent numbers 9,427,531 and 9,566,395, incorporated herein by reference in their entireties.

In one embodiment, the antibody molecule or antigen-binding fragment inhibits, reduces or neutralizes or block fibrosis in a subject. The subject can be a mammal, e.g., a primate, preferably a higher primate, e.g., a human (e.g., a patient having, or at risk of having, a disorder described herein). In one embodiment, the subject is in need of inhibiting, reducing, neutralizing or blocking fibrosis or a fibrotic condition.

According to the invention, "subject" means a human subject or human patient.

According to the present invention "inhibiting a disease" means inhibiting or slowing the full development of a disease. In several examples, inhibiting a disease refers to lessening symptoms of a fibrosis, such as the formation of scar tissue or an increase in range of motion or a decrease in pain. In some embodiments, inhibiting a disease refers to reducing or slowing progression of fibrosis. "Treatment" refers to a therapeutic intervention that ameliorates a sign or symptom of a disease or pathological condition related to the disease, such as the fibrosis, and/or slowing progression of the disease or pathological condition related to the disease, such as fibrosis.

According to certain embodiments of the present invention, "fibrosis" is the formation or development of excess fibrous connective tissue in an organ or tissue as a reparative or reactive process, as opposed to a formation of fibrous tissue as a normal constituent of an organ or tissue. In certain embodiments of the present invention fibrosis is the accumulation of fibrous connective tissue in and around inflamed or damaged tissue, which can lead to permanent scarring, organ malfunction and, ultimately, death. Fibrosis is the major histopathologic feature of a variety of clinical conditions and a pathological feature of most chronic inflammatory diseases. Skin and lungs are susceptible to fibrosis, but also the heart, liver, kidneys, and the gastrointestinal tract.

In embodiments of the invention, an inhibitor is disclosed that specifically targets the pathogenesis of fibrosis, having anti-fibrotic activity or effects, by targeting CD248, which is a pro-fibrotic mediator, preferably by targeting CD248 so as to reduce or prevent binding of IGFBP5 thereto

Exemplary fibrotic conditions are scleroderma, idiopathic pulmonary fibrosis, morphea, fibrosis as a result of Graft-Versus-Host Disease (GVHD), keloid and hypertrophic scar, and subepithelial fibrosis, endomyocardial fibrosis, uterine fibrosis, myelofibrosis, retroperitoneal fibrosis, nephrogenic systemic fibrosis, scarring after surgery, asthma, cirrhosis/liver fibrosis, aberrant wound healing, glomerulonephritis, and multifocal fibrosclerosis.

"Idiopathic Pulmonary Fibrosis" is a condition also known as cryptogenic fibrosing alveolitis (CFA) that is a chronic, progressive form of lung disease characterized by fibrosis of the supporting framework (interstitium) of the lungs. By definition, the term is used only when the cause of the pulmonary fibrosis is unknown ("idiopathic"). When lung tissue from patients with IPF is examined under a microscope by a pathologist, it shows a characteristic set of histologic/pathologic features known as usual interstitial pneumonia (UIP). UIP is characterized by progressive scarring of both lung that involves the supporting framework (interstitium) of the lung.

"Scleroderma" is a chronic autoimmune disease characterized by fibrosis (or hardening), vascular alterations, and autoantibodies. There are two major forms, one is a limited systemic scleroderma form that includes limited cutaneous scleroderma-mainly affects the hands, arms and face, although pulmonary hypertension is frequent. While, diffuse cutaneous scleroderma (or systemic sclerosis) is rapidly progressing and affects a large area of the skin and one or more internal organs, frequently the kidneys, esophagus, heart and lungs. Systemic scleroderma in both of its forms can be fatal. Other forms of scleroderma include systemic scleroderma, which lacks skin changes with systemic manifestations and two localized form that affect the skin, but not internal organs: morphea and linear scleroderma. The disclosed antibodies can be used to treat any form of scleroderma.

The antibodies and antigen-binding fragments disclosed herein can be used to treat fibrosis occurring in several disease conditions. The antibodies and antigen-binding fragments disclosed herein may decrease fibrosis that has already occurred, resolving existing fibrosis, and/or may decrease the rate or amount of additional fibrosis. In several examples, the antibodies and antigen-binding fragments are of use to decrease fibrosis in pathogenic processes, such as in a subject

Thus, in several embodiments, the methods include administering to a subject a therapeutically effective amount of one or more of the antibodies disclosed herein, or antigen-binding fragment thereof in order to decrease fibrosis. Any of the antibodies disclosed herein can be used to decrease fibrosis. In some embodiments, the antibodies and antigen-binding fragments thereof can be administered as a unit dose.

Suitable subjects include those with a fibrosis of the skin or lungs, but fibrosis of any tissue can be treated using the methods disclosed herein. In one example, the subject has scleroderma. In other examples, the subject has idiopathic pulmonary fibrosis, morphea, fibrosis as a result of Graft-Versus-Host Disease (GVHD), a keloid or hypertrophic scar, subepithelial fibrosis, endomyocardial fibrosis, uterine fibrosis, myelofibrosis, retroperitoneal fibrosis, nephrogenic systemic fibrosis, scarring after surgery, asthma, cirrhosis/liver fibrosis, aberrant wound healing, glomerulonephritis, and multifocal fibrosclerosis, liver fibrosis, kidney fibrosis.

In further examples, the methods are used to treat the systemic form of scleroderma, such as limited cutaneous scleroderma or diffuse cutaneous scleroderma (or systemic sclerosis). The methods can be used to treat the localized form of scleroderma, including morphea and linear scleroderma.

The methods can include selecting a subject in need of treatment, such as a subject with a fibrotic disease, such as scleroderma, idiopathic pulmonary fibrosis, morphea, a keloid scar, a hypertrophic scar, or subepithelial fibrosis. In exemplary applications, compositions are administered to a subject having a fibrotic disease, such as scleroderma, idiopathic pulmonary fibrosis, morphea, a keloid scar, a hypertrophic scar, or subepithelial fibrosis, or any of the disorders listed above, in an amount sufficient to reduce the fibrosis. Amounts effective for this use will depend upon the severity of the disease, the general state of the patient's health, and the robustness of the patient's immune system. In one example, a therapeutically effective amount of the compound is that which provides either subjective relief of a symptom(s) or an objectively identifiable improvement as noted by the clinician or other qualified observer.

A method is provided herein for decreasing skin thickness. The method includes administering a therapeutically effective amount of an anti-CD248 antibody, such as those disclosed herein, or antigen-binding fragment thereof, thereby decreasing skin thickness. In another embodiment, and methods is provided for decreasing lung fibrosis. The method includes administering a therapeutically effective amount of an antibody, or antigen-binding fragment thereof, thereby decreasing skin thickness. Any of the antibody disclosed herein can be used in these methods.

Methods are provided herein for decreasing COL1A1 or FN1 expression, such as transforming growth factor (TGF)-β +/- IGFBP5 induced COL1A1 or FN1 expression. The method includes contacting a cell with an effective amount of an anti-CD248 antibody, such as those disclosed herein, or antigen-binding fragment thereof, thereby decreasing COL1A1 or FN1 expression. The methods can be practiced *in vivo* or *in vitro.* In some embodiments, the methods include comparing the amount of COL1A1 or FN1 expression produced by a cell contacted with an antibody to a control. The control can be a standard value, or the amount of COL1A1 or FN1 produced by a cell not contacted with the antibody, such as a cell contacted with a carrier.

An antibody herein disclosed, or antigen-binding fragment thereof, can be administered by any means known to one of skill in the art either locally or systemically, such as by intradermal, intrathecal, intramuscular, subcutaneous, intraperitoneal or intravenous injection, but even oral, nasal, transdermal or anal administration is contemplated. In one embodiment, administration is by subcutaneous, intradermal, or intramuscular injection. In another embodiment, administration is by intraperitoneal or intrathecal administration. To extend the time during which the antibody is available to stimulate a response, the antibody or antigen-binding fragment thereof can be provided as an implant, an oily injection, or as a particulate system. The particulate system can be a microparticle, a microcapsule, a microsphere, a nanocapsule, or similar particle, (see, e.g., Banga, supra).

For treatment of the skin, a therapeutically effective amount of the antibody or antigen-binding fragment thereof can be locally administered to the affected area of the skin, such as in the form of an ointment (RGA Jones and A Marino, "Targeted localized use of therapeutic antibodies: a review of non-systemic, topical and oral applications, Crit. Rev. Biotechnol. 36(3):506-520 (2016).

In one embodiment, the ointment is an entirely homogenous semi-solid external agent with a firmness appropriate for easy application to the skin. Such an ointment can include fats, fatty oils, lanoline, Vaseline, paraffin, wax, hard ointments, resins, plastics, glycols, higher alcohols, glycerol, water or emulsifier and a suspending agent. Using these ingredients as a base, a decoy compound can be evenly mixed. Depending on the base, the mixture can be in the form of an oleaginous ointment, an emulsified ointment, or a water-soluble ointment oleaginous ointments use bases such as plant and animal oils and fats, wax, Vaseline and liquid paraffin. Emulsified ointments are comprised of an oleaginous substance and water, emulsified with an emulsifier. They can take either an oil-in-water form (O/W) or a water-in-oil-form (W/O). The oil-in-water form (O/W) can be a hydrophilic ointment. The water-in-oil form (W/O) initially lacks an aqueous phase and can include hydrophilic Vaseline and purified lanoline, or it can contain a water-absorption ointment (including an aqueous phase) and hydrated lanoline. A water-soluble ointment can contain a completely water-soluble Macrogol base as its main ingredient.

Pharmaceutically acceptable carriers include a petroleum jelly, such as VASELINE^{®}, wherein the petroleum jelly contains 5% stearyl alcohol, or petroleum jelly alone, or petroleum jelly containing liquid paraffin. Such carriers enable pharmaceutical compositions to be prescribed in forms appropriate for consumption, such as tablets, pills, sugar-coated agents, capsules, liquid preparations, gels, ointments, syrups, slurries, and suspensions. When locally administered into cells in an affected area or a tissue of interest, the at least one C-terminal endostatin polypeptide, or polynucleotide encoding the peptide can be administered in a composition that contains a synthetic or natural hydrophilic polymer as the carrier. Examples of such polymers include hydroxypropyl cellulose and polyethylene glycol. One or more antibody can be mixed with a hydrophilic polymer in an appropriate solvent. The solvent is then removed by methods such as air-drying, and the remainder is then shaped into a desired form (for example, a sheet) and applied to the target site. Formulations containing such hydrophilic polymers keep well as they have a low water-content. At the time of use, they absorb water, becoming gels that also store well. In the case of sheets, the firmness can be adjusted by mixing a polyhydric alcohol with a hydrophilic polymer similar to those above, such as cellulose, starch and its derivatives, or synthetic polymeric compounds. Hydrophilic sheets thus formed can be used. A therapeutically effective amount of one or more antibodies can also be incorporated into bandages and dressings.

For administration by inhalation, the antibody or antigen-binding fragment thereof can be conveniently delivered in the form of an aerosol spray presentation from pressurized packs or a nebulizer, with the use of a suitable propellant, such as dichlorodifluoromethane, trichlorofluoromethane, dichlorotetrafluoroethane, carbon dioxide or other suitable gas. In the case of a pressurized aerosol, the dosage unit can be determined by providing a valve to deliver a metered amount. Capsules and cartridges for use in an inhaler or insufflator can be formulated containing a powder mix of the compound and a suitable powder base such as lactose or starch.

In some embodiments, the antibody or antigen-binding fragment thereof can be administered by inhalation. For example, it can be administered in an aerosolized form, such as using a nebulizer or a metered dose inhaler. Technologies of use include micropump nebulizers (such as the AEROGEN GO^{®} system), jet nebulizers designed to produce large fine particle fractions (such as the PARI LC STAR^{®}), jet nebulizers developing less shear during atomization (such as the HUDSON MICROMIST^{®}), and ultrasonic nebulizers (such as the DeVilbiss ULTRA-NEB^{®}).

The antibody or antigen-binding fragment thereof can be dissolved in a carrier, such as saline, and atomized using the devices above. The associated aerosols can be collected using a NEXT GENERATION IMPACTOR^{®} (NGI) (MSP Corp., Shoreview, Minn.), which uses a series of aerodynamic stages to separate and collect the aerosol into separate fractions based on droplet size. Since droplet size is the primary determinant of deposition location in the lungs, this device allows us to specifically isolate the portion of the liquid aerosol that will deposit in the small airways and alveoli.

Aerosol particle size is often expressed in terms of mass median aerodynamic diameter (MMAD), a parameter that is based on particle size, shape, and density. For a spherical particle, MMAD is equal to MMD (p<1/2>), in which MMD is mass median diameter and r is the bulk density. For a non-spherical particle, MMAD is equal to MMD (p/x)<1/2>, in which X is the shape factor. Thus, particles with larger than unit density will have actual diameters smaller than their MMAD.

The site of particle deposition within the respiratory tract is demarcated based on particle size. In one example, particles of about 1 to about 500 microns are utilized, such as particles of about 25 to about 250 microns, or about 10 to about 25 microns are utilized. In other embodiments, particles of about 1 to 50 microns are utilized. For use in a metered dose inhaler, for administration to lungs particles of less than about 10 microns, such as particles of about 2 to about 8 microns, such as about 1 to about 5 microns, such as particles of 2 to 3 microns, can be utilized.

A therapeutically effect amount of an antibody or antigen-binding fragment thereof can be administered in the pharmaceutically acceptable carrier. Pharmacologically acceptable carriers (e.g., physiologically or pharmaceutically acceptable carriers) are well known in the art, and include, but are not limited to buffered solutions as a physiological pH (e.g. from a pH of about 7.0 to about 8.0, or at a pH of about 7.4). One specific, non-limiting example of a physiologically compatible buffered solution is phosphate buffered saline. Other pharmacologically acceptable carriers include penetrants, which are particularly suitable for pharmaceutical formulations that are intended to be topically applied (for example in the application of surgical wounds to promote healing).

The pharmacological compositions disclosed herein facilitate the use of at least one antibody or antigen-binding fragment thereof, either in vivo or ex vivo, to decrease fibrosis. Such a composition can be suitable for delivery of the active ingredient to any suitable subject, and can be manufactured in a manner that is itself known, e.g., by means of conventional mixing, dissolving, granulating, emulsifying, encapsulating, entrapping or lyophilizing processes. Pharmacological compositions can be formulated in a conventional manner using one or more pharmacologically (e.g., physiologically or pharmaceutically) acceptable carriers, as well as optional auxiliaries that facilitate processing of the active compounds into preparations which can be used pharmaceutically. Proper formulation is dependent upon the route of administration chosen. Thus, for injection, the active ingredient can be formulated in aqueous solutions. For transmucosal administration, penetrants appropriate to the barrier to be permeated are used in the formulation. Such penetrants are generally known in the art.

For oral administration, the active ingredient can be combined with carriers suitable for incorporation into tablets, pills, capsules, liquids, gels, syrups, slurries, suspensions and the like. The active ingredient can be formulated for parenteral administration by injection, such as by bolus injection or continuous infusion. Such compositions can take such forms as suspensions, solutions or emulsions in oily or aqueous vehicles, and can contain formulatory agents such as suspending, stabilizing and/or dispersing agents. Other pharmacological excipients are known in the art.

Other delivery systems can include time-release, delayed release or sustained release delivery systems. Such systems can avoid repeated administrations of the compositions of the invention described above, increasing convenience to the subject and the physician. Many types of release delivery systems are available and known to those of ordinary skill in the art. They include polymer-based systems such as poly(lactide-glycolide), copolyoxalates, polycaprolactones, polyesteramides, polyorthoesters, polyhydroxybutyric acid, and polyanhydrides. Microcapsules of the foregoing polymers containing drugs are described in, for example, U.S. Pat. No. 5,075,109. Delivery systems also include non-polymer systems, such as lipids including sterols such as cholesterol, cholesterol esters and fatty acids or neutral fats such as mono- di- and triglycerides; hydrogel release systems; silastic systems; peptide based systems; wax coatings; compressed tablets using conventional binders and excipients; partially fused implants; and the like. Specific examples include, but are not limited to: (a) erosional systems in which the at least one C-terminal endostatin polypeptide, or polynucleotide encoding the peptide is contained in a form within a matrix such as those described in U.S. Pat. Nos. 4,452,775; 4,667,014; 4,748,034; 5,239,660; and 6,218,371 and (b) diffusional systems in which an active component permeates at a controlled rate from a polymer such as described in U.S. Pat. Nos. 3,832,253 and 3,854,480. In addition, pump-based hardware delivery systems can be used, some of which are adapted for implantation.

Use of a long-term sustained release implant may be particularly suitable for treatment of chronic conditions, such as scleroderma. Long-term release, as used herein, means that the implant is constructed and arranged to deliver therapeutic levels of the active ingredient for at least 30 days, and preferably 60 days. Long-term sustained release implants are well known to those of ordinary skill in the art and include some of the release systems described above. These systems have been described for use with oligodeoxynucleotides (see U.S. Pat. No. 6,218,371). For use in vivo, nucleic acids and peptides are preferably relatively resistant to degradation (such as via endo- and exo-nucleases). Thus, modifications, such as the inclusion of a C-terminal amide, can be used.

The therapeutically effective amount of the antibody or antigen-binding fragment thereof will be dependent on the antibody that is utilized, the subject being treated, the severity and type of the affliction, and the manner of administration. For example, a therapeutically effective amount of an antibody can vary from about 0.01 µg per kilogram (kg) body weight to about 1 g per kg body weight, such as about 1 µg to about 5 mg per kg body weight, or about 5 µg to about 1 mg per kg body weight. The exact dose is readily determined by one of skill in the art based on the potency of the specific compound the age, weight, sex and physiological condition of the subject.

Single or multiple administrations of the compositions are administered depending on the dosage and frequency as required and tolerated by the subject. In one embodiment, the dosage is administered once as a bolus, but in another embodiment can be applied periodically until a therapeutic result is achieved. Generally, the dose is sufficient to treat or ameliorate symptoms or signs of disease without producing unacceptable toxicity to the subject. Systemic or local administration can be utilized.

In a further method, an additional agent is administered. In one example, this administration is sequential. In other examples, the additional agent is administered simultaneously with the antibody.

For the treatment of scleroderma, examples of additional agents that can be used with the antibody or antigen-binding fragment thereof include nifedipine, amlodipine, diltiazem, felodipine, or nicardipine. An investigational drug Gleevec, is also used for the treatment of scleroderma. Gleevec or other tyrosine kinase inhibitors can be used with the antibodies or antigen-binding fragment thereof disclosed herein. Patients with lung involvement of scleroderma benefit from oxygen therapy; the C-terminal endostatin polypeptides disclosed herein can be administered with this therapy.

For the treatment of fibrosis of the skin and scleroderma, additional agents of use are d-penicillamine, colchicine, Relaxin, steroids, and cyclosporine. In some embodiments, the additional agent is SAR100842 (see Allanore et al., Arthr. Rheum. 70(10):1634-1643 (2018)). C-terminal endostatin polypeptides also can be used in combination with immunosuppressive agents. Additionally, the antibodies can be used with methotrexate, cyclophosphamide, azathioprine, mycophenolate, glitazones, endothelin receptor antagonists, or Fulvestrant (ICI-182,780).

The disclosure is illustrated by the following non-limiting Examples.

### EXAMPLES

### Example 1

### Monoclonal antibodies development

Based on our novel demonstration (Example 6, below) that CD248 is an IGFBP5 receptor, a discovery campaign was undertaken to identify anti-CD248 antibodies that prevent binding of IGFBP5 and, preferably, thereby inhibit fibrosis mediated by IGFBP5. Monoclonal anti-CD248 antibodies were enriched from a naive human Fab phage-display library using either soluble recombinant human and mouse CD248 protein or human embryonal kidney (HEK) 293 cells expressing human CD248 after transient transfection (i.e. CD248-positive cells), according to five different selection strategies, identified as S1-S2-S3-S4-S5, and summarized in Table 1.

First, unspecific antibody-phages were cleared from the library through a negative selection cycle. For that purpose, the library was incubated in presence of: a) Streptavidin-Beads/surface-immobilized BSA or b) Streptavidin-Beads/surface-immobilized BSA and untransfected human embryonal kidney (HEK) 293 cells (i.e. CD248-negative cells) or c) untransfected HEK 293 cells (CD248-negative cells). Antibody-phages that bound to either Streptavidin-Beads/surface-immobilized BSA or CD248-negative cells were discarded from further selection. After this step, the cleared library was selected for target antigen-specific antibodies. Depending on the strategy, recombinant biotinylated human or mouse CD248 (produced internally) was added to the library preparations or CD248-positive cells. For strategies S1-S2-S3, antibody-phages that bound to the biotinylated target antigen were captured and recovered from the solution using magnetic streptavidin beads. The beads were washed multiple times with a BSA solution (PBS containing 0.05% Tween20 and 2% BSA) in order to remove unspecific or weakly bound antibody-phage particles. Antigen-specific antibody phage were eluted from the beads by Trypsin treatment and rescued by E. coli infection. After short propagation, the bacteria were coinfected with M13K07 helper phage and antibody-phage amplification was induced. The amplified phages were used for two more selection cycles as described above. For strategies S4-S5, antibody-phages that bound to the CD248-positive cells were separated from the non-binding phage in solution by centrifugation. The cells were washed multiple times to remove unspecific or weakly bound antibody-phage particles. Antigen-specific antibody phage were rescued by transferring the cells into an E. coli culture for phage-infection. An overview of the antibody selection strategies is given in Table 1:

| **Table 1: Overview of the antibodies selection strategies** | | | | |
|---|---|---|---|---|
| **Selection Strategy** | **Negative Selection (cycles 1-3)** | **Positive Selection (cycle 1)** | **Positive Selection (cycle 2)** | **Positive Selection (cycle 3)** |
| S1 | Streptavidin-Beads, surface immobilized BSA | Human CD248 | Human CD248 | Human CD248 |
| S2 | Streptavidin-Beads, surface immobilized BSA | Mouse CD248 | Mouse CD248 | Mouse CD248 |
| S3 | Streptavidin-Beads, surface immobilized BSA | Human CD248 | Mouse CD248 | Human CD248 |
| S4 | Streptavidin-Beads, surface immobilized BSA, CD248-negative cells | Human CD248 | Human CD248-positive cells | Human CD248-positive cells |
| | | Mouse CD248 | | |
| S5 | CD248-negative cells | Human CD248-positive cells | Mouse CD248-positive cells | Human CD248-positive cells |

At the end of the discovery process, each selection output was screened for antigen-specific antibodies and the binding characteristics of monoclonal antibody clones was analysed. For this purpose, 384 single clones for each selection strategy (1,920 total clones) were used for Fab antibody production in the form of E-Coli supernatants. 1152 Fab clones isolated from Strategy S1-S2-S3 were screened for specific antigen binding by ELISA on: a) human recombinant CD248 (internally produced), b) human recombinant CD248 (commercial, R&D Systems, Cat# 7855-CD-050), c) murine recombinant CD248 (internally produced). Fabs were also screened for binding to BSA (2% solution), as negative antigen. Antibody clones were defined as antigen specific "hits" if: a) the ELISA signal on positive antigens was higher than 0.1, b) the ELISA signal on the negative antigen was lower than 0.1; c) Signal/Noise (S/N) ratio (e.g. ratio between both positive and negative antigens signal) was higher than 3. In total 178 clones were isolated with a specific antigen binding (-15% hit rate): 127/178 bound both forms of human recombinant CD248 (internally produced and commercial), 22/178 were cross-reactive with human (internally produced and commercial) and mouse recombinant CD248, 29/178 bound mouse recombinant CD248; 53/178 were derived from S1 strategy, 55/178 were derived from S2 strategy, 70/178 were derived from S3 strategy.

768 Fab clones isolated from Strategy S4-S5 were screened for specific antigen binding by flow cytometry using HEK 293-tranfected with human CD248 (i.e. CD248-positive cells). Antibody clones were defined as antigen specific "hit" if signal/noise ratio between CD248-positive cells and CD248-negative cells was higher than 3. In total 564 clones were isolated with a specific antigen binding (-73% hit rate): 316/564 were derived from S4 strategy and 248/564 were derived from S5 strategy.

DNA sequence analysis of the 742 antigen-specific antibody clones selected after the screening process (178 selected from the S1-S2-S3 strategy + 564 selected from the S4-S5 strategy) was performed. A bioinformatic analysis was carried out to identify similar/non-similar antibodies based on the complementary determining region (CDR) diversity (clones with ≤ 5 amino acid differences spread across all the three heavy-chain CDR and all the three light-chain CDR were considered similar and expected to bind to the same/similar epitope). At the end of sequencing process, 259 antibodies with unique sequences were identified, yielding overall to a ~35% uniqueness rate, belonging to 14 CDR clusters; 62/259 were derived from S1-S2-S3 strategy and 197/259 were derived from S4-S5 strategy.

Following the identification of 259 CD248-specific antibodies with unique sequences, 93 clones were cherry picked based on S/N ratio registered during the screening, soluble Fab antibodies were produced in E. Coli and the non-purified Fab culture supernatant was tested in ELISA to calculate its capacity to inhibit the interaction of recombinant human CD248 and recombinant human IGFBP5 in vitro. Fab bacterial supernatants were incubated with biotinylated CD248 (internally produced) in solution at a ratio 1:1. Hence the mix of Fab-CD248 was added to surface-immobilized human IGFBP5 and detection of CD248 interaction with IGFBP5 was performed via Streptavidin-HRP. For 10 antibodies a small inhibitory effect of the binding between CD248 and IGFBP5 was detectable. The inhibition was at least 10-fold higher than aspecific inhibition produced by the negative control antibody used (Palivizumab). To better estimate the inhibitory binding potential of the 10 selected anti-CD248 Fab antibodies, they were converted into full IgG4, carrying the S228P/L235E mutations, that were produced in HEK 293 cells, purified with Affinity chromatography based on Protein A and resuspended in Sodium Phosphate 78 mM/Sodium Chloride 0.136 M. The protein concentration was determined by UV/VIS spectrometry and purity was checked by SDS PAGE. All the 10 antibodies could be successfully produced in the IgG4 format, carrying the S228/L235 mutations, except the YU1023-E03, that was scarcely detectable in SDS-PAGE and showed suboptimal concentration (<0.1 mg/ml). The list of the 10 antibodies and their concentration is provided in Table 2.

| **Table 2: List of antibodies produced in the IgG4 format, all carrying the S228/L235E mutations** | | |
|---|---|---|
| **Antibody** | **Conc [mg/ml]** | **Total mg produced** |
| YU1022-D08 | 1.37 | 3.98 |
| YU1022-E09 | 2.15 | 6.25 |
| YU1023-A02 | 0.14 | 0.42 |
| YU1023-B03 | 0.69 | 2.00 |
| YU1023-E03 | 0.07 | 0.21 |
| YU1024-G01 | 1.63 | 4.72 |
| YU1024-H02 | 2.92 | 8.45 |
| YU1025-B07 | 1.80 | 5.21 |
| YU1025-C01 | 2.19 | 6.35 |
| YU1027-G07 | 2.65 | 7.68 |

Purified IgG4 antibodies were then tested for the capacity to inhibit the binding of human recombinant CD248 to human recombinant IGFBP5, as described above. The half maximal inhibitory concentration (IC50) was calculated for the six antibodies showing the highest inhibition capacity. Results are summarized in Table 3.

| **Table 3: List of antibodies produced in the IgG4 format, all carrying the S228/L235E mutations** | | |
|---|---|---|
| **Antibody** | **Panning strategy** | **IC 50 [µg/ml]** |
| YU1024-G01 | S3 | 4.7 |
| YU1023-E03 | S2 | 17.4 |
| YU1025-B07 | S4 | 19.2 |
| YU1023-A02 | S3 | 20.8 |
| YU1022-D08 | S3 | 41.6 |
| YU1023-B03 | S2 | 47.6 |

The sequences of the 6 novel anti-CD248 antibodies showing inhibitory activity of the binding between human recombinant CD248 and human recombinant IGFBP5 are reported in Tables 4-15. CDR sequences in Tables 4 and 5 are according to Kabat definition.

| **Table 4: VH CDR Sequences of exemplified antibodies** | | | |
|---|---|---|---|
| **Antibody** | **VH CDR1** | **VH CDR2** | **VH CDR3** |
| YU1022-D08 | SYAMS (SEQ ID No. 1) | AISGSGGSTYYADSVKG (SEQ ID No. 4) | LTGTT (SEQ ID No. 7) |
| YU1023-A02 | SGGYYWS (SEQ ID No. 2) | YIYYSGSTYYNPSLKS (SEQ ID No. 5) | VRGRSATYFDY (SEQ ID No. 8) |
| YU1023-B03 | SYAMS (SEQ ID No. 1) | AISGSGGSTYYADSVKG (SEQ ID No. 4) | RSGGFDY (SEQ ID No. 9) |
| YU1023-E03 | SYAMH (SEQ ID No. 3) | VISYDGSNKYYADSVKG (SEQ ID No. 6) | SIAAAGTRWFDP (SEQ ID No. 10) |
| YU1024-G01 | SYAMH (SEQ ID No. 3) | VISYDGSNKYYADSVKG (SEQ ID No. 6) | NNHPRGERLPQY (SEQ ID No. 11) |
| YU1025-B07 | SYAMH (SEQ ID No. 3) | VISYDGSNKYYADSVKG (SEQ ID No. 6) | TKARWFGVTTYMDV (SEQ ID No. 12) |

| **Table 5: VL CDR sequences of exemplified antibodies** | | | |
|---|---|---|---|
| **Antibody** | **VL CDR1** | **VL CDR2** | **VL CDR3** |
| YU1022-D08 | KSSRSVLYSSNNKNYLA (SEQ ID No. 13) | WASTRES (SEQ ID No. 19) | QQYYSTPYS (SEQ ID No. 24) |
| YU1023-A02 | TGSSSNIGAGYDVR (SEQ ID No. 14) | ANNNRPS (SEQ ID No. 20) | QSYD!SLSGSL (SEQ ID No. 25) |
| YU1023-B03 | RSSQSLLHSNGYNYLD (SEQ ID No. 15) | FTSNRAS (SEQ ID No. 21) | MQSIQLPRT (SEQ ID No. 26) |
| YU1023-E03 | RASQGISSWLA (SEQ ID No. 16) | AASSLQS (SEQ ID No. 22) | QQANSFPLT (SEQ ID No. 27) |
| YU1024-G01 | RASQSVGSYLN (SEQ ID No. 17) | VASRLQS (SEQ ID No. 23) | QQSYSTPLT (SEQ ID No. 28) |
| YU1025-B07 | KSSQSVLYSSNNKNYLA (SEQ ID No. 18) | WASTRES (SEQ ID No. 19) | QQYYSTPPT (SEQ ID No. 29) |

| **Table 6: VH amino acid sequences of exemplified antibodies** | |
|---|---|
| **Antibody** | **AA of VH** |
| YU1022-D08 | EVQLLESGGGLVQPGGSLRLSCAASGFTFSSYAMSWVRQAPGKGL EWVSAISGSGGSTYYADSVKGRFTISRDNSKNTLYLQMNSLRAEDT AVYYCAKLTGTTWGQGTLVTVSS (SEQ ID No. 30) |
| YU1023-A02 | QVQLVESGPGLVKPSQTLSLTCTVSGGSISSGGYYWSWIRQHPGKG LEWIGYIYYSGSTYYNPSLKSRVTISVDTSKNQFSLKLSSVTAADTAV YYCARVRGRSATYFDYWGQGTTVTVSS (SEQ ID No. 31) |
| YU1023-B03 | EVQLLESGGGLVQPGGSLRLSCAASGFTFSSYAMSWVRQAPGKGL EWVSAISGSGGSTYYADSVKGRFTISRDNSKNTLYLQMNSLRAEDT AVYYCAARSGGFDYWGQGTLVTVSS (SEQ ID No. 32) |
| YU1023-E03 | EVQLLESGGGVVQPGRSLRLSCAASGFTFSSYAMHWVRQAPGKGL EWVAVISYDGSNKYYADSVKGRFTISRDNSKNTLYLQMNSLRAEDTA VYYCARSIAAAGTRWFDPWGQGTLVTVSS (SEQ ID No. 33) |
| YU1024-G01 | QVQLQESGGGVVQPGRSLRLSCAASGFTFSSYAMHWVRQAPGKG LEWVAVISYDGSNKYYADSVKGRFTISRDNSKNTLYLQMNSLRAEDT AVYYCARNNHPRGERLPQYWGQGTLVTVSS (SEQ ID No. 34) |
| YU1025-B07 | QVQLVQSGGGVVQPGRSLRLSCAASGFTFSSYAMHWVRQAPGKG LEWVAVISYDGSNKYYADSVKGRFTISRDNSKNTLYLQMNSLRAEDT AVYYCARTKARWFGVTTYMDVWGKGTLVTVSS (SEQ ID No. 35) |

| **Table 7: VL amino acid sequences of exemplified antibodies** | |
|---|---|
| **Antibody** | **AA of VL** |
| YU1022-D08 | DIVMTQSPDSLAVSLGERATINCKSSRSVLYSSNNKNYLAWYQQK PGQPPKLLIYWASTRESGVPDRFSGSGSGTDFTLTISSLQAEDVAV YYCQQYYSTPYSFGQGTKVEIK (SEQ ID No. 36) |
| YU1023-A02 | QSVLTQPPSVSGAPGQRVTISCTGSSSNIGAGYDVRWYQQLPGT APKLLIYANNNRPSGVPDRFSGSKSGTSASLAITGLQTEDEADYYC QSYDISLSGSLFGGGTKLTVL (SEQ ID No. 37) |
| YU1023-B03 | DVVMTQSPLSLPVTPGEPASISCRSSQSLLHSNGYNYLDWYVQKP GQSPQRLIYFTSNRASGVPDRFSGSGSGTDFTLKISRVEAEDVGV YYCMQSIQLPRTFGQGTKVEIK (SEQ ID No. 38) |
| YU1023-E03 | DIQLTQSPSSVSASVGDRVTITCRASQGISSWLAWYQQKPGKAPK LLIYAASSLQSGVPSRFSGSGSGTDFTLTISSLQPEDFATYYCQQA NSFPLTFGGGTKLEIK (SEQ ID No. 39) |
| YU1024-G01 | DIQLTQSPSSLSASVGDRVTITCRASQSVGSYLNWYQHKPGKAPK LLIYVASRLQSGVPSRFSGSGSGTDFTLTINSLQPEDFATYYCQQS YSTPLTFGGGTKVEIK (SEQ ID No. 40) |
| YU1025-B07 | DIVMTQSPDSLAVSLGERATINCKSSQSVLYSSNNKNYLAWYQQK PGQPPKLLIYWASTRESGVPDRFSGSGSGTDFTLTISSLQAEDVAV YYCQQYYSTPPTFGQGTKVEIK(SEQ ID No. 41) |

CDR definitions are also provided using annotation tool from http://www.abysis.org/ based on full VH and VL amino acid sequences as defined in Tables 4 and 5.

For example, the VH or VL amino acid sequence of any antibody disclosed herein is plugged into the annotation tool and Kabat defined CDR sequences, or IMGT, or Chothia, or AbM or Contact defined CDR sequences are provided. Using the "All, side by side" feature, defined CDR sequences are provided.

Table 8 shows such sequences related to YU1022-D08 VH and VL, Table 9 shows such sequences related to YU1023-A02 VH and VL, Table 10 shows such sequences related to YU1023-B03 VH and VL, Table 11 shows sequences related to YU1023-E03 VH and VL, Table 12 shows sequences related to YU1024-G01 VH and VL, Table 13 shows sequences related to YU1025-B07 VH and VL.

| **Table 8**: YU1022-D08 VH and VL CDRs according to different definitions as sorted by inserting aa VH (SEQ ID No. 30) and VL (SEQ. ID No. 36) sequence in the www.abysis.org annotation tool | | | | |
|---|---|---|---|---|
| **Region** | **Definition** | **Sequence Fragment** | **Residues** | **Length** |
| CDR-L1 | Chothia | KSSRSVLYSSNNKNYL A-(SEQ ID No. 13) | 24 - 40 | 17 |
| | AbM | KSSRSVLYSSNNKNYL A-(SEQ ID No. 13) | 24 - 40 | 17 |
| | Kabat | KSSRSVLYSSNNKNYL A-(SEQ ID No. 13) | 24 - 40 | 17 |
| | Contact | -LYSSNNKNYLAWY (SEQ ID No. 59) | 30 - 42 | 13 |
| | IMGT | --- RSVLYSSNNKNY-(SEQ ID No. 60) | 27 - 38 | 12 |
| CDR-L2 | Chothia | ----WASTRES (SEQ ID No. 19) | 56 - 62 | 7 |
| | AbM | ----WASTRES (SEQ ID No. 19) | 56 - 62 | 7 |
| | Kabat | ----WASTRES (SEQ ID No. 19) | 56 - 62 | 7 |
| | Contact | LLIYWASTRE-(SEQ ID No. 61) | 52 - 61 | 10 |
| | IMGT | ----WA----- | 56 - 57 | 2 |
| CDR-L3 | Chothia | QQYYSTPYS (SEQ ID No. 24) | 95 - 103 | 9 |
| | AbM | QQYYSTPYS (SEQ ID No. 24) | 95 - 103 | 9 |
| | Kabat | QQYYSTPYS (SEQ ID No. 24) | 95 - 103 | 9 |
| | Contact | QQYYSTPY-(SEQ ID No. 63) | 95 - 102 | 8 |
| | IMGT | QQYYSTPYS (SEQ ID No. 24) | 95 - 103 | 9 |
| CDR-H1 | Chothia | GFTFSSY-(SEQ ID No. 64) | 26 - 32 | 7 |
| | AbM | GFTFSSYAMS (SEQ ID No. 65) | 26 - 35 | 10 |
| | Kabat | -----SYAMS (SEQ ID No. 1) | 31 - 35 | 5 |
| | Contact | ----SSYAMS (SEQ ID No. 66) | 30 - 35 | 6 |
| | IMGT | GFTFSSY A-(SEQ ID No. 67) | 26 - 33 | 8 |
| CDR-H2 | Chothia | -----SGSGGS-(SEQ ID No. 68) | 52 - 57 | 6 |
| | AbM | ---AISGSGGSTY-(SEQ ID No. 69) | 50 - 59 | 10 |
| | Kabat | -AISGSGGSTYYADSVK G (SEQ ID No. 4) | 50 - 66 | 17 |
| | Contact | WVSAISGSGGSTY----- -(SEQ ID No. 70) | 47 - 59 | 13 |
| | IMGT | ----ISGSGGST (SEQ ID No. 71) | 51 - 58 | 8 |
| CDR-H3 | Chothia | --LTGTT (SEQ ID No. 7) | 99 - 103 | 5 |
| | AbM | --LTGTT (SEQ ID No. 7) | 99 - 103 | 5 |
| | Kabat | --LTGTT (SEQ ID No. 7) | 99 - 103 | 5 |
| | Contact | AKLTGT-(SEQ ID No. 72) | 97 - 102 | 6 |
| | IMGT | AKLTGTT (SEQ ID No. 73) | 97 - 103 | 7 |

| **Table 9:** YU1023-A02 VH and VL CDRs according to different definitions as sorted by inserting aa VH (SEQ ID No. 31) and VL (SEQ. ID No. 37) sequence in the www.abysis.org annotation tool | | | | |
|---|---|---|---|---|
| **Region** | **Definition** | **Sequence fragment** | **Residues** | **Length** |
| CDR-L1 | Chothia | TGSSSNIGAGYDVR --(SEQ ID No. 14) | 23 - 36 | 14 |
| | AbM | TGSSSNIGAGYDVR --(SEQ ID No. 14) | 23 - 36 | 14 |
| | Kabat | TGSSSNIGAGYDVR --(SEQ ID No. 14) | 23 - 36 | 14 |
| | Contact | ------IGAGYDVRWY (SEQ ID No. 74) | 29 - 38 | 10 |
| | IMGT | ---SSNIGAGYO-(SEQ ID No. 75) | 26 - 34 | 9 |
| CDR-L2 | Chothia | ----ANNNRPS (SEQ ID No. 20) | 52 - 58 | 7 |
| | AbM | ----ANNNRPS (SEQ ID No. 20) | 52 - 58 | 7 |
| | Kabat | ----ANNNRPS (SEQ ID No. 20) | 52 - 58 | 7 |
| | Contact | LLIYANNNRP-(SEQ ID No. 76) | 48 - 57 | 10 |
| | IMGT | ----AN----- | 52 - 53 | 2 |
| CDR-L3 | Chothia | QSYD!SLSGSL (SEQ ID No. 25) | 91 - 101 | 11 |
| | AbM | QSYD!SLSGSL (SEQ ID No. 25) | 91 - 101 | 11 |
| | Kabat | QSYD!SLSGSL (SEQ ID No. 25) | 91 - 101 | 11 |
| | Contact | QSYDISLSGS-(SEQ ID No. 77) | 91 - 100 | 10 |
| | IMGT | QSYD!SLSGSL (SEQ ID No. 25) | 91 - 101 | 11 |
| CDR-H 1 | Chothia | GGSISSGGY-(SEQ ID No. 78) | 26 - 34 | 9 |
| | AbM | GGSISSGGYYWS (SEQ ID No. 79) | 26 - 37 | 12 |
| | Kabat | ----- SGGYYWS (SEQ ID No. 2) | 31 - 37 | 7 |
| | Contact | ---- SSGGYYWS (SEQ ID No. 88) | 30 - 37 | 8 |
| | IMGT | GGSISSGGYY-(SEQ ID No. 89) | 26 - 35 | 10 |
| CDR-H2 | Chothia | -----YYSGS-(SEQ ID No. 90) | 54 - 58 | 5 |
| | AbM | ---YIYYSGSTY-(SEQ ID No. 91) | 52 - 60 | 9 |
| | Kabat | -YIYYSGSTYYNPSL KS (SEQ ID No. 5) | 52 - 67 | 16 |
| | Contact | WIGYIYVSGSTY----- --(SEQ ID No. 92) | 49 - 60 | 12 |
| | IMGT | ----IYYSGST (SEQ ID No. 93) | 53 - 59 | 7 |
| CDR-H3 | Chothia | --VRGRSATYFDY (SEQ ID No. 8) | 100 - 110 | 11 |
| | AbM | --VRGRSATYFDY (SEQ ID No. 8) | 100 - 110 | 11 |
| | Kabat | --VRGRSATYFDY (SEQ ID No. 8) | 100 - 110 | 11 |
| | Contact | ARVRGRSATYFD-(SEQ ID No. 94) | 98 - 109 | 12 |
| | IMGT | ARVRGRSATYFDY (SEQ ID No. 95) | 98 - 110 | 13 |

| **Table 10:** YU1023-B03 VH and VL CDRs according to different definitions as sorted by inserting aa VH (SEQ ID No. 32) and VL (SEQ. ID No. 38) sequence in the www.abysis.org annotation tool | | | | |
|---|---|---|---|---|
| **Region** | **Definition** | **Sequence fragment** | **Residues** | **Length** |
| CDR-L1 | Chothia | RSSQSLLHSNGYNYL D-- (SEQ ID No. 15) | 24 - 39 | 16 |
| | AbM | RSSQSLLHSNGYNYL D-- (SEQ ID No. 15) | 24 - 39 | 16 |
| | Kabat | RSSQSLLHSNGYNYL D-- (SEQ ID No. 15) | 24 - 39 | 16 |
| | Contact | -LHSNGYNYLDWY (SEQ ID No. 96) | 30 - 41 | 12 |
| | IMGT | ---QSLLHSNGYNY-(SEQ ID No. 97) | 27 - 37 | 11 |
| CDR-L2 | Chothia | ---- FTSNRAS (SEQ ID No. 21) | 55 - 61 | 7 |
| | AbM | ----FTSNRAS (SEQ ID No. 21) | 55 - 61 | 7 |
| | Kabat | ----FTSNRAS (SEQ ID No. 21) | 55 - 61 | 7 |
| | Contact | RLIYFTSNRA-(SEQ ID No. 98) | 51 - 60 | 10 |
| | IMGT | ----FT----- | 55 - 56 | 2 |
| CDR-L3 | Chothia | MQSIQLPRT (SEQ ID No. 26) | 94 - 102 | 9 |
| | AbM | MQSIQLPRT (SEQ ID No. 26) | 94 - 102 | 9 |
| | Kabat | MQSIQLPRT (SEQ ID No. 26) | 94 - 102 | 9 |
| | Contact | MQSIQLPR-(SEQ ID No. 100) | 94 - 101 | 8 |
| | IMGT | MQSIQLPRT (SEQ ID No. 26) | 94 - 102 | 9 |
| CDR-H1 | Chothia | GFTFSSY-(SEQ ID No. 64) | 26 - 32 | 7 |
| | AbM | GFTFSSYAMS (SEQ ID No. 65) | 26 - 35 | 10 |
| | Kabat | -----SYAMS (SEQ ID No. 1) | 31 - 35 | 5 |
| | Contact | ----SSYAMS (SEQ ID No. 66) | 30 - 35 | 6 |
| | IMGT | GFTFSSYA-(SEQ ID No. 67) | 26 - 33 | 8 |
| CDR-H2 | Chothia | -----SGSGG-(SEQ ID No. 68) | 52 - 57 | 6 |
| | AbM | ---AISGSGGSTY-(SEQ ID No. 69) | 50 - 59 | 10 |
| | Kabat | -AISGSGGSTYYADSV KG (SEQ ID No. 4) | 50 - 66 | 17 |
| | Contact | VWSAISGSGGSTY---- ---(SEQ ID No. 70) | 47 - 59 | 13 |
| | IMGT | ----ISGSGGST (SEQ ID No. 71) | 51 - 58 | 8 |
| CDR-H3 | Chothia | --RSGGFDY (SEQ ID No. 9) | 99 - 105 | 7 |
| | AbM | --RSGGFDY (SEQ ID No. 9) | 99 - 105 | 7 |
| | Kabat | --RSGGFDY (SEQ ID No. 9) | 99 - 105 | 7 |
| | Contact | AARSGGFD-(SEQ ID No. 1 01) | 97 - 104 | 8 |
| | IMGT | AARSGGFDY (SEQ ID No. 102) | 97 - 105 | 9 |

| **Table 11:** YU1023-E03 VH and VL CDRs according to different definitions as sorted by inserting aa VH (SEQ ID No. 33) and VL (SEQ. ID No. 39) sequence in the www.abysis.org annotation tool | | | | |
|---|---|---|---|---|
| **Region** | **Definition** | **Sequence fragment** | **Residues** | **Length** |
| CDR-L1 | Chothia | RASQGISSWLA-(SEQ ID No. 16) | 24 - 34 | 11 |
| | AbM | RASQGISSWLA-(SEQ ID No. 16) | 24 - 34 | 11 |
| | Kabat | RASQGISSWLA-(SEQ ID No. 16) | 24 - 34 | 11 |
| | Contact | ------SSWLAWY (SEQ ID No. 103) | 30 - 36 | 7 |
| | IMGT | ---QGISSW-(SEQ ID No. 104) | 27 - 32 | 6 |
| CDR-L2 | Chothia | ----AASS LQS (SEQ ID No. 22) | 50 - 56 | 7 |
| | AbM | ----AASS LQS (SEQ ID No. 22) | 50 - 56 | 7 |
| | Kabat | ----AASS LQS (SEQ ID No. 22) | 50 - 56 | 7 |
| | Contact | LLIYAASSLQ-(SEQ ID No. 105) | 46 - 55 | 10 |
| | IMGT | ---- AA ----- | 50 - 51 | 2 |
| CDR-L3 | Chothia | QQANSFPLT (SEQ ID No. 27) | 89 - 97 | 9 |
| | AbM | QQANSFPLT (SEQ ID No. 27) | 89 - 97 | 9 |
| | Kabat | QQANSFPLT (SEQ ID No. 27) | 89 - 97 | 9 |
| | Contact | QQANSFPL-(SEQ ID No. 107) | 89 - 96 | 8 |
| | IMGT | QQANSFPLT (SEQ ID No. 27) | 89 - 97 | 9 |
| CDR-H1 | Chothia | GFTFSSY-(SEQ ID No. 108) | 26 - 32 | 7 |
| | AbM | GFTFSSYAMH (SEQ ID No. 109) | 26 - 35 | 10 |
| | Kabat | -----SYAMH (SEQ ID No. 3) | 31 - 35 | 5 |
| | Contact | ----SSYAM H (SEQ ID No. 110) | 30 - 35 | 6 |
| | IMGT | GFTFSSYA-(SEQ ID No. 111) | 26 - 33 | 8 |
| CDR-H2 | Chothia | -----SYDGSN -------- (SEQ ID No. 112) | 52 - 57 | 6 |
| | AbM | ---VISYDGSNKY-(SEQ ID No. 113) | 50 - 59 | 10 |
| | Kabat | -VISYDGSNKYYADSV KG (SEQ ID No. 6) | 50 - 66 | 17 |
| | Contact | WVAVISYDGSNKY---- --- (SEQ ID No. 114) | 47 - 59 | 13 |
| | IMGT | ----ISYDGSNK (SEQ ID No. 115) | 51 - 58 | 8 |
| CDR-H3 | Chothia | --SIAAAGTRWFDP (SEQ ID No. 1 0) | 99 - 110 | 12 |
| | AbM | --SIAAAGTRWFDP (SEQ ID No. 1 0) | 99 - 110 | 12 |
| | Kabat | --SIAAAGTRWFDP (SEQ ID No. 1 0) | 99 - 110 | 12 |
| | Contact | ARSIAAAGTRWFD-(SEQ ID No. 116) | 97 - 109 | 13 |
| | IMGT | ARSIAAAGTRWFDP (SEQ ID No. 117) | 97 - 110 | 14 |

| **Table 12:** YU1024-G01 VH and VL CDRs according to different definitions as sorted by inserting aa VH (SEQ ID No. 34) and VL (SEQ. ID No. 40) sequence in the www.abysis.org annotation tool | | | | |
|---|---|---|---|---|
| **Region** | **Definition** | **Sequence fragment** | **Residues** | **Length** |
| CDR-L1 | Chothia | RASQSVGSYLN-(SEQ ID No. 17) | 24 - 34 | 11 |
| | AbM | RASQSVGSYLN-(SEQ ID No. 17) | 24 - 34 | 11 |
| | Kabat | RASQSVGSYLN-(SEQ ID No. 17) | 24 - 34 | 11 |
| | Contact | ------GSYLNWY (SEQ ID No. 118) | 30 - 36 | 7 |
| | IMGT | ---QSVGSY-(SEQ ID No. 119) | 27 - 32 | 6 |
| CDR-L2 | Chothia | ----VASRLQS (SEQ ID No. 23) | 50 - 56 | 7 |
| | AbM | ----VAS RLQS (SEQ ID No. 23) | 50 - 56 | 7 |
| | Kabat | ----VAS RLQS (SEQ ID No. 23) | 50 - 56 | 7 |
| | Contact | LLIYVASRLQ-(SEQ ID No. 120) | 46 - 55 | 10 |
| | IMGT | ----VA----- | 50 - 51 | 2 |
| CDR-L3 | Chothia | QQSYSTPLT (SEQ ID No. 28) | 89 - 97 | 9 |
| | AbM | QQSYSTPLT (SEQ ID No. 28) | 89 - 97 | 9 |
| | Kabat | QQSYSTPLT (SEQ ID No. 28) | 89 - 97 | 9 |
| | Contact | QQSYSTPL-(SEQ ID No. 122) | 89 - 96 | 8 |
| | IMGT | QQSYSTPLT (SEQ ID No. 28) | 89 - 97 | 9 |
| CDR-H1 | Chothia | GFTFSSY-(SEQ ID No. 108) | 26 - 32 | 7 |
| | AbM | GFTFSSYAMH (SEQ ID No. 109) | 26 - 35 | 10 |
| | Kabat | -----SYAMH (SEQ ID No. 3) | 31 - 35 | 5 |
| | Contact | ----S SYAM H (SEQ ID No. 110) | 30 - 35 | 6 |
| | IMGT | GFTFSSY A-(SEQ ID No. 111) | 26 - 33 | 8 |
| CDR-H2 | Chothia | -----SYDGSN -------- (SEQ ID No. 112) | 52 - 57 | 6 |
| | AbM | ---VISYDGSNKY-(SEQ ID No. 113) | 50 - 59 | 10 |
| | Kabat | -VISYDGSNKYYADSV KG (SEQ ID No. 6) | 50 - 66 | 17 |
| | Contact | WVAVISYDGSNKY---- --- (SEQ ID No. 114) | 47 - 59 | 13 |
| | IMGT | ----ISYDGSNK (SEQ ID No. 115) | 51 - 58 | 8 |
| CDR-H3 | Chothia | --NNHPRGERLPQY (SEQ ID No. 11) | 99 - 110 | 12 |
| | AbM | --NNHPRGERLPQY (SEQ ID No. 11) | 99 - 110 | 12 |
| | Kabat | --NNHPRGERLPQY (SEQ ID No. 11) | 99 - 110 | 12 |
| | Contact | ARNNHPRGERLPQ-(SEQ ID No. 123) | 97 - 109 | 13 |
| | IMGT | ARNNHPRGERLPQY (SEQ ID No. 124) | 97 - 110 | 14 |

| **Table 13:** YU1025-B07 VH and VL CDRs according to different definitions as sorted by inserting aa VH (SEQ ID No. 35) and VL (SEQ. ID No. 41) sequence in the www.abysis.org annotation tool | | | | |
|---|---|---|---|---|
| **Region** | **Definition** | **Sequence fragment** | **Residues** | **Length** |
| CDR-L1 | Chothia | KSSQSVLYSSNNKNY LA-- (SEQ ID No. 18) | 24 - 40 | 17 |
| | AbM | KSSQSVLYSSNNKNY LA-- (SEQ ID No. 18) | 24 - 40 | 17 |
| | Kabat | KSSQSVLYSSNNKNY LA-- (SEQ ID No. 18) | 24 - 40 | 17 |
| | Contact | -LYSSNNKNYLAWY (SEQ ID No. 125) | 30 - 42 | 13 |
| | IMGT | --- QSVLYSSNNKNY --- - (SEQ ID No. 126) | 27 - 38 | 12 |
| CDR-L2 | Chothia | ----WASTRES (SEQ ID No. 19) | 56 - 62 | 7 |
| | AbM | ----WASTRES (SEQ ID No. 19) | 56 - 62 | 7 |
| | Kabat | ----WASTRES (SEQ ID No. 19) | 56 - 62 | 7 |
| | Contact | LLIYWASTRE-(SEQ ID No. 61) | 52 - 61 | 10 |
| | IMGT | ----WA----- | 56 - 57 | 2 |
| CDR-L3 | Chothia | QQYYSTPPT (SEQ ID No. 29) | 95 - 103 | 9 |
| | AbM | QQYYSTPPT (SEQ ID No. 29) | 95 - 103 | 9 |
| | Kabat | QQYYSTPPT (SEQ ID No. 29) | 95 - 103 | 9 |
| | Contact | QQYYSTPP-(SEQ ID No. 127) | 95 - 102 | 8 |
| | IMGT | QQYYSTPPT (SEQ ID No. 29) | 95 - 103 | 9 |
| CDR-H1 | Chothia | GFTFSSY-(SEQ ID No. 108) | 26 - 32 | 7 |
| | AbM | GFTFSSYAMH (SEQ ID No. 109) | 26 - 35 | 10 |
| | Kabat | -----SYAMH (SEQ ID No. 3) | 31 - 35 | 5 |
| | Contact | ----SSYAMH (SEQ ID No. 110) | 30 - 35 | 6 |
| | IMGT | GFTFSSYA-(SEQ ID No. 111) | 26 - 33 | 8 |
| CDR-H2 | Chothia | ----- SYDGSN -------- (SEQ ID No. 112) | 52 - 57 | 6 |
| | AbM | ---VISYDGSNKY-(SEQ ID No. 113) | 50 - 59 | 10 |
| | Kabat | -VISYDGSNKYYADSV KG (SEQ ID No. 6) | 50 - 66 | 17 |
| | Contact | WVAVISYDGSNKY---- --- (SEQ ID No. 114) | 47 - 59 | 13 |
| | IMGT | ----ISYDGSNK (SEQ ID No. 115) | 51 - 58 | 8 |
| CDR-H3 | Chothia | TKARWFGVTTYMDV (SEQ ID No. 12) | 99 - 112 | 14 |
| | AbM | TKARWFGVTTYMDV (SEQ ID No. 12) | 99 - 112 | 14 |
| | Kabat | TKARWFGVTTYMDV (SEQ ID No. 12) | 99 - 112 | 14 |
| | Contact | ARTKARWFGVTTYM D- (SEQ ID No. 128) | 97 - 111 | 15 |
| | IMGT | ARTKARWFGVTTYM DV (SEQ ID No. 129) | 97 - 112 | 16 |

| **Table 14: VH Framework Regions amino acid sequences of exemplified antibodies** | | | | |
|---|---|---|---|---|
| **Antibody** | **FR1-VH** | **FR2-VH** | **FR3-VH** | **FR4-VH** |
| YU1022-D08 | EVQLLESGGGL VQPGGSLRLSC AASGFTFS (SEQ ID No. 130) | WVRQAP GKGLEW VS (SEQ ID No. 135) | RFTISRDNSKNTL YLQMNSLRAEDT AVYYCAK (SEQ ID No. 138) | WGQGTLVTVS S (SEQ ID No. 140) |
| YU1023-A02 | QVQLVESGPGL VKPSQTLSLTCT VSGGSIS (SEQ ID No. 131) | WIRQHPG KGLEWIG (SEQ ID No. 136) | RVTISVDTSKNQF SLKLSSVTAADTA VYYCAR (SEQ ID No. 139) | WGQGTTVTVS S (SEQ ID No. 141) |
| YU1023-B03 | EVQLLESGGGL VQPGGSLRLSC AASGFTFS (SEQ ID No. 130) | WVRQAP GKGLEW VS (SEQ ID No. 135) | RFTISRDNSKNTL YLQMNSLRAEDT AVYYCAA (SEQ ID No. 152) | WGQGTLVTVS S (SEQ ID No. 140) |
| YU1023-E03 | EVQLLESGGGV VQPGRSLRLSC AASGFTFS (SEQ ID No. 132) | WVRQAP GKGLEW VA (SEQ ID No. 137) | RFTISRDNSKNTL YLQMNSLRAEDT AVYYCAR (SEQ ID No. 153) | WGQGTLVTVS S (SEQ ID No. 140) |
| YU1024-G01 | QVQLQESGGGV VQPGRSLRLSC AASGFTFS (SEQ ID No. 133) | WVRQAP GKGLEW VA (SEQ ID No. 137) | RFTISRDNSKNTL YLQMNSLRAEDT AVYYCAR (SEQ ID No. 153) | WGQGTLVTVS S (SEQ ID No. 140) |
| YU1025-B07 | QVQLVQSGGGV VQPGRSLRLSC AASGFTFS (SEQ ID No. 134) | WVRQAP GKGLEW VA (SEQ ID No. 137) | RFTISRDNSKNTL YLQMNSLRAEDT AVYYCAR (SEQ ID No. 153) | WGKGTLVTVS S (SEQ ID No. 142) |

| **Table 15: VL Framework Regions amino acid sequences of exemplified antibodies** | | | | |
|---|---|---|---|---|
| **Antibo dy** | **FR1-VL** | **FR2-VL** | **FR3-VL** | **FR4-VL** |
| YU102 2-D08 | DIVMTQSPDSLAV SLGERATINC (SEQ ID No. 143) | WYQQKPG QPPKLLIY (SEQ ID No. 148) | GVPDRFSGSGSGT DFTLTISSLQAEDV AVYYC (SEQ ID No. 81) | FGQGTKVEIK (SEQ ID No. 87) |
| YU102 3-A02 | QSVLTQPPSVSG APGQRVTISC (SEQ ID No. 144) | WYQQLPGT APKLLIY (SEQ ID No. 149) | GVPDRFSGSKSGT SASLAITGLQTEDE ADYYC (SEQ ID No. 82) | FGGGTKLTVL (SEQ ID No. 99) |
| YU102 3-B03 | DVVMTQSPLSLP VTPGEPASISC (SEQ ID No. 145) | WYVQKPG QSPQRLIY (SEQ ID No. 150) | GVPDRFSGSGSGT DFTLKISRVEAEDV GVYYC (SEQ ID No. 83) | FGQGTKVEIK (SEQ ID No. 87) |
| YU102 3-E03 | DIQLTQSPSSVSA SVGDRVTITC (SEQ ID No. 146) | WYQQKPG KAPKLLIY (SEQ ID No. 151) | GVPSRFSGSGSGT DFTLTISSLQPEDF ATYYC (SEQ ID No. 84) | FGGGTKLEIK (SEQ ID No. 106) |
| YU102 4-G01 | DIQLTQSPSSLSA SVGDRVTITC (SEQ ID No. 147) | WYQHKPG KAPKLLIY (SEQ ID No. 80) | GVPSRFSGSGSGT DFTLTINSLQPEDF ATYYC (SEQ ID No. 85) | FGGGTKVEIK (SEQ ID No. 121) |
| YU102 5-B07 | DIVMTQSPDSLAV SLGERATINC (SEQ ID No. 143) | WYQQKPG QPPKLLIY (SEQ ID No. 148) | GVPDRFSGSGSGT DFTLTISSLQAEDV AVYYC (SEQ ID No. 86) | FGQGTKVEIK (SEQ ID No. 87) |

| **Table 16: VH nucleotide sequences of exemplified antibodies** | |
|---|---|
| **Antibody** | **DNA of VH** |
| YU1022-D08 | GAGGTACAACTGCTCGAGTCAGGCGGGGGGCTCGTCCAACC AGGGGGATCTTTGCGCCTGTCTTGTGCTGCTTCTGGTTTTACA TTTTCCAGCTATGCGATGAGCTGGGTACGGCAGGCCCCAGGC AAAGGACTCGAGTGGGTGTCAGCGATTAGTGGTAGTGGTGGG TCTACATATTACGCTGACAGCGTGAAGGGGCGGTTTACAATTT CTCGAGACAACTCTAAGAACACCCTGTACCTTCAAATGAACAG TCTGAGAGCCGAGGATACTGCTGTTTATTATTGCGCGAAACTT ACGGGTACAACTTGGGGACAGGGGACACTCGTCACTGTCTCT TCA (SEQ ID No. 42) |
| YU1023-A02 | CAGGTCCAATTGGTGGAAAGTGGGCCGGGCCTTGTCAAACCT TCTCAGACACTCTCTCTCACCTGTACTGTTTCAGGTGGAAGCA TAAGTAGTGGTGGTTATTACTGGTCATGGATTCGCCAACATCC |
| | TGGGAAGGGACTTGAATGGATCGGTTACATTTATTATTCCGGT TCCACCTACTACAATCCGAGTCTCAAGTCTCGCGTAACGATAT CCGTTGATACTTCTAAGAATCAGTTCAGTCTTAAATTGAGTTCA GTCACCGCCGCCGATACAGCGGTCTATTATTGTGCAAGGGTC CGAGGCCGGTCCGCTACATACTTTGACTACTGGGGTCAGGGG ACAACCGTAACAGTATCTTCA (SEQ ID No. 43) |
| YU1023-B03 | GAGGTCCAACTTCTCGAAAGCGGCGGTGGCCTGGTGCAGCC GGGTGGTTCACTGCGGTTGTCCTGTGCTGCAAGCGGATTTAC ATTTAGCAGCTATGCCATGTCATGGGTACGACAGGCACCCGG AAAGGGCCTTGAGTGGGTATCCGCAATTTCAGGCTCAGGCGG CAGCACCTATTATGCCGATAGCGTTAAGGGAAGATTTACCATC AGCCGAGATAATTCTAAAAATACACTTTACCTTCAAATGAACAG TTTGCGGGCTGAGGATACTGCAGTGTATTATTGCGCTGCGCG ATCTGGTGGGTTTGACTATTGGGGGCAGGGCACCTTGGTAAC AGTTAGTTCT (SEQ ID No. 44) |
| YU1023-E03 | GAGGTACAACTCTTGGAAAGCGGCGGCGGTGTAGTACAGCC GGGTAGAAGTCTCCGACTGAGCTGCGCCGCTTCTGGATTCAC ATTTTCCTCCTATGCGATGCATTGGGTCAGACAGGCCCCTGG GAAAGGGTTGGAATGGGTAGCTGTTATTAGTTATGATGGAAGC AATAAGTATTACGCAGATTCCGTTAAAGGCCGATTCACCATTT CACGAGATAATTCAAAGAACACTCTTTATTTGCAGATGAACTCA CTCAGAGCCGAAGATACGGCGGTGTACTACTGTGCTAGAAGC ATTGCGGCTGCAGGGACGCGCTGGTTCGATCCGTGGGGTCA GGGAACTCTGGTTACAGTAAGCAGC (SEQ ID No. 45) |
| YU1024-G01 | CAGGTCCAACTGCAGGAGTCAGGTGGAGGCGTCGTACAACCA GGGCGAAGTCTGCGACTTTCATGCGCTGCAAGCGGTTTCACA TTCTCCTCATACGCGATGCACTGGGTGCGGCAGGCTCCCGGC AAGGGCCTGGAATGGGTCGCGGTTATCTCTTACGACGGAAGC AACAAATACTACGCCGACAGTGTGAAGGGCCGATTCACAATAA GTCGGGATAATTCAAAAAACACATTGTACTTGCAAATGAACAG CCTGCGCGCGGAGGACACTGCTGTCTACTACTGTGCCCGAAA TAATCATCCCAGAGGTGAACGCTTGCCGCAGTATTGGGGTCA GGGTACACTCGTTACTGTATCCTCT (SEQ ID No. 46) |
| YU1025-B07 | CAGGTTCAGTTGGTTCAATCTGGAGGCGGAGTCGTCCAACCT GGTCGGTCACTTAGGCTTTCTTGTGCGGCATCAGGTTTCACCT TCTCATCTTACGCGATGCACTGGGTACGGCAAGCGCCTGGAA AAGGTCTGGAGTGGGTCGCCGTGATTTCCTACGACGGGAGTA ATAAATATTATGCAGACAGTGTCAAAGGCAGGTTCACGATTTC AAGGGACAACTCTAAGAATACCTTGTACCTTCAAATGAACTCC CTGCGAGCGGAAGATACGGCGGTTTATTACTGTGCTCGGACG AAAGCCCGCTGGTTTGGAGTGACCACGTATATGGACGTTTGG GGGAAAGGTACTCTTGTGACTGTCTCTTCT (SEQ ID No. 47) |

| **Table 17: VL nucleotide sequences of exemplified antibodies** | |
|---|---|
| **Antibody** | **DNA of VL** |
| YU1022-D08 | GATATAGTAATGACACAATCACCTGACAGCCTCGCAGTTAGCC TCGGAGAGCGGGCGACCATTAACTGCAAGAGCAGTCGGTCA GTTCTCTACTCAAGTAACAATAAGAACTACCTCGCATGGTACC AACAAAAGCCCGGCCAACCCCCAAAGTTGCTCATATACTGGG CATCTACCCGGGAAAGCGGGGTGCCGGATCGCTTTTCCGGCA GCGGTAGCGGCACTGACTTCACCCTCACGATTTCTTCTCTTCA GGCAGAGGACGTAGCGGTATATTACTGTCAGCAGTATTATAG CACTCCTTATTCTTTCGGGCAGGGGACCAAAGTGGAAATTAAG (SEQ ID No. 48) |
| YU1023-A02 | CAATCTGTCTTGACTCAGCCTCCCAGTGTTTCTGGAGCGCCG GGCCAGAGGGTGACTATTAGTTGCACCGGAAGTTCAAGTAAT ATAGGTGCGGGTTATGACGTTCGATGGTATCAGCAGCTCCCC GGAACTGCGCCTAAGCTCCTTATATACGCAAATAACAACAGGC CCAGTGGGGTACCAGATCGGTTCTCTGGGTCTAAAAGCGGCA CATCCGCGAGCCTTGCAATCACAGGGTTGCAAACTGAAGATG AAGCTGACTATTATTGCCAAAGCTACGACATAAGTCTTAGCGG CTCCCTGTTCGGCGGGGGGACTAAATTGACCGTTCTG (SEQ ID No. 49) |
| YU1023-B03 | GATGTTGTAATGACTCAATCCCCTCTGAGCCTTCCCGTTACAC CGGGGGAGCCAGCCTCAATAAGCTGCCGATCCTCTCAATCTC TCCTGCATAGTAATGGCTACAATTATCTTGATTGGTATGTTCAG AAACCAGGTCAGTCACCTCAAAGACTCATCTATTTCACTAGCA ACCGAGCCAGCGGGGTACCGGACCGATTTTCAGGCAGCGGC TCAGGTACAGATTTCACCCTTAAGATTAGCAGAGTGGAAGCCG AGGATGTTGGGGTCTATTACTGTATGCAATCCATACAGCTGCC GAGGACGTTTGGACAAGGAACTAAAGTGGAGATAAAG (SEQ ID No. 50) |
| YU1023-E03 | GATATTCAGCTCACCCAGTCCCCTAGCAGTGTTTCAGCCTCTG TTGGGGACCGCGTCACAATAACTTGTCGCGCGTCACAAGGAA TAAGCTCATGGCTGGCCTGGTATCAACAAAAGCCAGGTAAAG CTCCCAAACTTCTTATATATGCCGCGTCATCTCTCCAGAGCGG GGTTCCTTCCCGATTTTCCGGTTCCGGTTCAGGCACGGACTT CACATTGACGATTAGTTCCTTGCAACCCGAAGATTTTGCAACA TATTACTGTCAACAAGCGAATAGCTTTCCGCTGACATTCGGTG GTGGCACGAAATTGGAGATTAAA (SEQ ID No. 51) |
| YU1024-G01 | GATATTCAGTTGACACAGAGTCCCTCCAGTTTGAGCGCAAGTG TAGGAGACAGAGTTACGATAACTTGTCGAGCCTCACAAAGTGT CGGGTCTTACTTGAATTGGTATCAGCACAAACCCGGCAAGGC ACCGAAGCTGTTGATATATGTGGCTAGTAGATTGCAGAGCGG GGTGCCTTCAAGGTTTTCAGGGTCCGGCTCTGGGACTGATTT TACGTTGACAATTAATAGCCTGCAACCGGAGGATTTCGCTACA TATTACTGCCAGCAGAGCTACTCAACGCCGCTCACATTTGGAG GCGGGACGAAGGTTGAGATTAAG (SEQ ID No. 52) |
| YU1025-B07 | GACATTGTAATGACACAGAGTCCTGACAGTCTCGCTGTATCTC TCGGAGAGCGGGCGACTATCAACTGTAAATCTTCACAATCAGT GCTTTATTCCTCTAACAACAAAAACTACCTTGCGTGGTATCAAC AAAAGCCTGGTCAGCCTCCTAAACTGCTGATATATTGGGCGTC CACTCGCGAATCCGGCGTTCCCGACCGATTTAGCGGTAGCGG ATCAGGCACGGATTTTACCCTCACCATATCATCCTTGCAGGCG GAGGATGTCGCTGTTTACTATTGCCAGCAATACTACTCAACCC CTCCCACCTTTGGACAAGGAACCAAGGTGGAGATAAAA (SEQ ID No. 53) |

| **Table 18: Constant region amino acid sequences** | |
|---|---|
| **Constant region** | **AA** |
| Human IgG4 heavy chain (wild type) | ASTKGPSVFPLAPCSRSTSESTAALGCLVKDYFPEPVTVSW NSGALTSGVHTFPAVLQSSGLYSLSSVVTVPSSSLGTKTYTC NVDHKPSNTKVDKRVESKYGPPCPSCPAPEFLGGPSVFLFP PKPKDTLMISRTPEVTCVVVDVSQEDPEVQFNWYVDGVEVH NAKTKPREEQFNSTYRVVSVLTVLHQDWLNGKEYKCKVSNK GLPSSIEKTISKAKGQPREPQVYTLPPSQEEMTKNQVSLTCL VKGFYPSDIAVEWESNGQPENNYKTTPPVLDSDGSFFLYSR LTVDKSRWQEGNVFSCSVMHEALHNHYTQKSLSLSLGK (SEQ ID No. 54) |
| Human IgG4 heavy chain with S228P/L235E mutations | ASTKGPSVFPLAPCSRSTSESTAALGCLVKDYFPEPVTVSW NSGALTSGVHTFPAVLQSSGLYSLSSVVTVPSSSLGTKTYTC NVDHKPSNTKVDKRVESKYGPPCPPCPAPEFEGGPSVFLFP PKPKDTLMISRTPEVTCVVVDVSQEDPEVQFNWYVDGVEVH NAKTKPREEQFNSTYRVVSVLTVLHQDWLNGKEYKCKVSNK GLPSSIEKTISKAKGQPREPQVYTLPPSQEEMTKNQVSLTCL VKGFYPSDIAVEWESNGQPENNYKTTPPVLDSDGSFFLYSR LTVDKSRWQEGNVFSCSVMHEALHNHYTQKSLSLSLGK (SEQ ID No. 62) |
| Human IgG2 heavy chain P01859 | ASTKGPSVFPLAPCSRSTSESTAALGCLVKDYFPEPVTVSW NSGALTSGVHTFPAVLQSSGLYSLSSVVTVPSSNFGTQTYT CNVDHKPSNTKVDKTVERKCCVECPPCPAPPVAGPSVFLFP PKPKDTLMISRTPEVTCVVVDVSHEDPEVQFNWYVDGVEVH NAKTKPREEQFNSTFRVVSVLTVVHQDWLNGKEYKCKVSN KGLPAPIEKTISKTKGQPREPQVYTLPPSREEMTKNQVSLTC LVKGFYPSDISVEWESNGQPENNYKTTPPMLDSDGSFFLYS KLTVDKSRWQQGNVFSCSVMHEALHNHYTQKSLSLSPGK (SEQ ID No. 55) |
| Human light chain, lambda 1 P0CG04 | GQPKANPTVTLFPPSSEELQANKATLVCLISDFYPGAVTVAW KADGSPVKAGVETTKPSKQSNNKYAASSYLSLTPEQWKSH RSYSCQVTHEGSTVEKTVAPTECS (SEQ ID No. 56) |
| Human light chain, lambda 2 P0DOY2 | GQPKAAPSVTLFPPSSEELQANKATLVCLISDFYPGAVTVAW KADSSPVKAGVETTTPSKQSNNKYAASSYLSLTPEQWKSHR SYSCQVTHEGSTVEKTVAPTECS (SEQ ID No. 57) |
| Human light chain, kappa P01834 |  (SEQ ID No. 58) |

### Example 2: Binding of antibodies to CD248-positive cells

The 6 antibodies selected after the screening process were tested for their ability to bind human and mouse CD248 expressed on the cell membrane of transiently transfected HEK 293 cells (i.e. CD248-positive cells). Binding to CD248 of anti-CD248 antibodies at concentration series was detected by flow cytometry, using a secondary anti-human IgG antibody. Mean Fluorescence Intensity (MFI) of antibody binding, representing the mean fluorescence signal given by the binding of anti-CD248 antibody to CD248 expressed by HEK 293 cells was quantified. As negative control, binding to an irrelevant antigen expressed on HEK 293 was assessed.

All six tested antibodies could efficiently bind both human and mouse CD248 expressed on HEK 293 cells. The binding to mouse CD248 expressed on cells confirmed that mice can be considered a relevant animal species for testing of the anti-CD248 monoclonal antibodies during preclinical development. Data obtained by flow cytometry were normalized and transformed for EC₅₀ calculation, that are shown in Table 19. The antibody that showed the highest affinity, in terms of EC₅₀, to human or murine CD248 expressed on the cell surface of HEK 293 cells was G01, while the worse binder was E03.

| **Table 19. EC50 calculated values for the six tested antibodies against human and mouse CD248 or an irrelevant antigen expressed on transiently transfected HEK 293** | | |
|---|---|---|
| Antibody | **Antigen** | **EC₅₀ [µg/ml]** |
| YU1022-D08 | Human CD248 | 0.02614 |
| YU1023-A02 | Human CD248 | 0.05229 |
| YU1023-B03 | Human CD248 | 0.0401 |
| YU1023-E03 | Human CD248 | 1.494 |
| YU1024-G01 | Human CD248 | 0.01208 |
| YU1025-B07 | Human CD248 | 0.02364 |
| YU1022-D08 | Human CD248 | 0.02385 |
| YU1023-A02 | Mouse CD248 | 0.03881 |
| YU1023-B03 | Mouse CD248 | 0.01843 |
| YU1023-E03 | Mouse CD248 | 0.8912 |
| YU1024-G01 | Mouse CD248 | ∼0.009854 |
| YU1025-B07 | Mouse CD248 | 0.02157 |
| YU1022-D08 | Irrelevant antigen | 1.184 |
| YU1023-A02 | Irrelevant antigen | 3.161 |
| YU1023-B03 | Irrelevant antigen | 3.659 |
| YU1023-E03 | Irrelevant antigen | 3.837 |
| YU1024-G01 | Irrelevant antigen | 2.108 |
| YU1025-B07 | Irrelevant antigen | 1.679 |

### Example 3. Specificity Assessment

The specificity of each antibody to bind human and mouse CD248 as compared to the binding to an irrelevant human antigen overexpressed on the cell membrane of HEK 293 cells after transient transfection was quantified. To this aim, data collected from the cell binding measurement assay as MFI of binding to the irrelevant antigen expressed on HEK 293 cells were analyzed: the MFI signal in the absence of an antibody was compared with the MFI signal read at the highest antibody concentration tested (10 µg/ml) on the cells overexpressing the irrelevant human antigen.

The highest specificity was observed for YU1023-B03 and YU1025-B07, while the lowest specificity was for YU1024-G01 (Figure 1). For reference, in Figure 1, it is represented the MFI signal obtained with anti-CD248 binding at the same concentration (10 µg/ml) on the cells overexpressing human or mouse CD248.

### Example 4. Affinity measurement

### Octet BLI-based analysis

To assess the binding affinity in more detail, affinity measurements were performed using Biomolecular Interaction Analysis performed by an Octet platform (BLItz System), which is a Biolayer Interferometry (BLI) platform. To establish the assay, the target monoclonal antibody (mAb, 2 identical antigen binding moieties, 2 µg/ml in 1% BSA containing 0.05% Tween 20) was immobilized via Fc on the via AHC biosensor and the interaction with the antigen human CD248 (internally produced) and mouse CD248 (internally produced) at different concentration were measured.

The affinity measurement of YU1022-D08, YU1023-A02, YU1023-B03, YU1024-G01, YU1025-B07 IgG4 mAb for the target human and murine CD248 are reported in the following Table 20.

| **Table 20: Affinity of and for the target human and murine CD248** | | |
|---|---|---|
| *Molecule ID* | *Human CD248 KD (M)* | *Murine CD248 KD (M)* |
| YU1022-D08 | 5.37E-09 | 1.19E-08 |
| YU1023-A02 | 9.94E-09 | 1.01E-08 |
| YU1023-B03 | 1.86E-08 | 7.99E-09 |
| YU1024-G01 | 3.78E-09 | 3.91E-09 |
| YU1025-B07 | 1.17E-08 | 1.42E-08 |

Newly generated anti-CD248 mAbs show good human antigen binding affinity with KD between 10⁻⁹M and 10⁻⁸M. The antibodies also show murine cross reactivity. This data confirmed that mice can be considered a relevant animal species for testing of the monoclonal antibodies during preclinical development.

### Example 5: Primary cell-based assay relevant to fibrosis (IPF)

Fibrosis is a process of continuous tissue repair characterized by the formation and deposition of fibrillary collagen-rich extracellular matrix (ECM) leading to a progressive remodeling in most tissues and organs. Formation of fibrotic scar tissue is essential to restore and maintain tissue and organ integrity during wound healing after injury. However, when the scarring mechanisms exacerbate after repetitive injury, chronic fibrogenesis results in a shift from supportive fibrotic tissue to scar tissue. This coincides with an increased number and activity of extracellular matrix producing cells, which finally leads to destruction of normal tissue/organ architecture and function.

In the lung, such as in Idiopathic Pulmonary Fibrosis disease (IPF), tissue scarring/fibrosis occurs. Tissue damage is followed by the activation of the immune system that induces release of several cytokines and growth factors including transforming growth factor beta-1 (TGFβ-1) that signals the tissue to repair. Mechanistically, epithelial to mesenchymal transition (EMT) is one of the major drivers of fibrosis, and approximately 30% of ECM-producing (myo-)fibroblasts derive from epithelial cells through EMT. During EMT, tightly organized epithelial cells lose expression of the tight junction marker E-cadherin and transform to mesenchymal cells with expression of the mesenchymal cell markers N-cadherin, vimentin and fibronectin. Furthermore, the cells gain migratory potential, enabling them to migrate throughout the tissue. Another important mechanism during fibrosis is fibroblast to myofibroblast transformation (FMT), when EMT-derived, resident and invading fibroblastoid cells are activated (e.g., by TGFβ-1) and start to differentiate towards myofibroblasts.

Myofibroblasts are a heterogeneous population derived from different progenitors, and are characterized by formation of alpha smooth muscle actin (αSMA) containing stress fibers and the expression and secretion of fibrillar collagen, fibronectin and additional ECM components. Increased production and accumulation of ECM result in a permanently damaged fibrotic tissue with an aberrant architecture unable to function properly. FMT and EMT in vitro assays are established models to identify and characterize anti-fibrotic compounds (see Weigle et al. J Biol Methods (2019) Vol. 6(2)). The in vitro cell model used to test the anti-fibrotic activity of anti-CD248 mAbs relies on the use of either epithelial lung cells derived from a human alveolar epithelial carcinoma cell line (AEC, A549) and human lung primary fibroblasts (HLF) derived from a control donor or from an IPF patient.

A549 alveolar epithelial cells were seeded on Day 0 in complete culture medium. After 24h cells were treated with human recombinant IGFBP5 (500 ng/ml) and human recombinant TGF-β1 (5 ng/ml). Anti-CD248 mAbs YU1022-D08, YU1023-A02 YU1023-B03, YU1024-G01 and YU1025-B07 were added at two concentrations (20 µg/ml and 40 µg/ml). After 48h, cells were collected for analysis. mRNA levels of Fibronectin *(FN1)* and collagen1 alpha 1 (*COL1A1*) were quantified by qRT-PCR. FN1 in culture supernatant was quantified by Western Blot and ELISA.

Treatment with YU1022-D08 mAb significantly reduced *FN1* and *COL1A1* mRNA expression as well as FN1 protein secretion (as quantified by both western blot and Elisa) induced by IGFBP5+TGFβ1 on A549 alveolar epithelial cells.

YU1023-A02 mAb significantly reduced *FN1* and *COL1A1* mRNA expression as well as FN1 protein secretion (quantified by western blot) induced by IGFBP5+TGFβ1 on A549 alveolar epithelial cells.

Treatment with YU1023-B03 significantly reduced *FN1* mRNA expression induced by IGFBP5+TGFβ1 on A549 alveolar epithelial cells.

Treatment with YU1024-G01 and YU1025-B07 mAbs significantly reduced *COL1A* mRNA expression induced by IGFBP5+TGFβ1 on A549 alveolar epithelial cells. Notably, YU1025-B07 (20 µg/ml) induced a significant increase in fibronectin secretion as quantified by ELISA, but this result was not confirmed at the highest dose (40 µg/ml) and with WB measurement (Figure 2).

Primary HLF derived from a control donor or from an IPF patient were seeded on Day 0 in complete culture medium. After 24h cell were treated with human recombinant IGFBP5 (500 ng/ml) and human recombinant TGF-β1 (1.25 ng/ml). Anti-CD248 mAbs YU1022-D08, YU1023-A02 YU1023-B03, YU1024-G01 and YU1025-B07 were added at two concentrations (20 µg/ml and 40 µg/ml). After 48h, cells were collected for analysis. mRNA levels of Fibronectin *(FN1)* were quantified by qRT-PCR.

Treatment with YU1023-B03 and YU1023-A02 significantly reduced FN1 mRNA expression in CTRL-HLF and IPF-HLF, respectively (Figure 3).

### Example 6: Identification of CD248 as IGFBP5 receptor

Prior to the research presented herein, the IGFBP5-receptor mediating IGFBP5 intracellular signalling and triggering of IGFBP5-profibrotic effect had not been identified yet. Hence, to identify the IGFBP5 receptor, the Ligand Receptor Capture (LRC)-TriCEPS^{™} technology has been employed. LRC-TriCEPS^{™} is an approach that enables the identification of cell surface targets and off-targets on living cells for a wide range of orphan ligands including small molecules, peptides, proteins, antibodies and viruses (Frei et al. Nature Protocol 2013,8). During the LRC-TriCEPS^{™} experiment, human recombinant IGFBP5 was coupled with the LRC-TriCEPS^{™} crosslinker followed by incubation with living human lung fibroblasts from n=3 different donors. Human lung fibroblasts were selected because they are known to be responsive to IGFBP5 (Nguyen et al. Front Endocrinol 2018, 9:601; Pilewski et al. Am J Path 2005,166) and hence to express an IGFBP5 receptor. In parallel, cells were incubated with control ligands: bovine serum albumin (BSA) was used as negative control and transferrin (TRFE) was used as positive control, for its known binding to the transferrin receptor expressed on the surface of human lung fibroblasts. During ligand-TriCEPS incubation, TriCEPS binds covalently to the glycans of random surface proteins, but also the targets of the ligand of interest and control ligands. Due to the ligand binding, the target proteins will be more heavily labelled compared to the random surface proteins. The crosslinked proteins were then purified and processed for analysis by mass-spectrometry-based proteomics (LC-MS/MS).

Enrichment of proteins in presence of IGFBP5 versus control ligands (BSA or TRFE) was expressed as fold change in log2 scale and statistically compared in terms of pvalue, which was then adjusted for multiple comparisons to control the experiment-wide false discovery rate.

The criteria to consider a protein as a candidate for interacting with IGFBP5 were the following:
1) Log 2 fold change > 2
2) Adjusted p value <0.05

As summarized in Table 21, after crosslinking the cell membrane of human lung fibroblasts from different donors (n=3), with TriCEPS^{™} bound to IGFBP5, the following proteins were found as significantly and consistently enriched against both negative control (BSA) and positive control (TRFE) and are proposed as candidate target/s:
- Reversion-inducing cysteine-rich protein with Kazal motifs (RECK)
- Chondroitin sulfate proteoglycan 4 (CSPG4)
- CD248.

CD44 was found borderline enriched and is considered a lower confidence interactor, but given its potential role in the fibrosis pathogenesis (Xia et al. Am J Physiol Lung Cell Mol Physiol 2021, 320) was included in the following biochemical characterization, together with RECK, CSPG4 and CD248.

**Table 21. Summary of identified enriched proteins along with enrichment (fold change in log2 scale and adjusted p value). General comparison against both controls (TRFE and BSA).**

| | | | **log2(FC)** | | **adj pValue** | |
|---|---|---|---|---|---|---|
| **Protein Name** | **Accession** | **nb of peptides** | **IGFBPS vs TRFE** | **IGFBP5 vs BSA** | **IGFBPS vs TRFE** | **IGFBP5 vs BSA** |
| IBPS | P24593 | 19 | -9.4 | -8.4 | 1.17E-04 | 1.35E-05 |
| GPC1 | P35052 | 1 | -5.5 | -5.9 | 3.01E-01 | 7.88E-03 |
| ADRB1 | P08588 | 1 | -4.1 | -4.9 | 4.41E-02 | 1.20E-03 |
| NRP1 | Q14786 | 1 | -3.7 | -3.8 | 8.68E-02 | 6.22E-03 |
| RECK | Q95980 | 7 | -2.3 | -4.3 | 3.28E-02 | 6.08E-04 |
| CSPG4 | Q6UVK1 | 4 | -2.3 | -2.2 | 2.88E-02 | 1.81E-03 |
| CD248 | Q9HCU0 | 5 | -1.8 | -2.2 | 9.03E-02 | 6.22E-03 |
| SNX18 | Q96RF 0 | 2 | -1.7 | -2.5 | 4.93E-01 | 1.71E-01 |
| CAVN1 | Q6NZI2 | 3 | -1.0 | -1.5 | 1.13E-01 | 9.64E-03 |
| ADT3 | P12236 | 4 | -1.0 | -1.6 | 1.68E-01 | 1.41E-02 |
| CD44 | P16070 | 6 | -0.6 | -0.8 | 1.88E-01 | 2.39E-02 |

The biochemical characterization of the interaction of human recombinant IGFBP5 with the identified candidate receptors was performed by ELISA using recombinant proteins as detailed in Table 22. Human CSPG4 was tested in two different formats: one as full length protein and one as fragment 1582-2224.

**Table 22. List of recombinant proteins used**

| **Protein** | **Cat #** | **Vendor** | **Source** | **Protein fragme nt length** | **Tag** | **Ref** |
|---|---|---|---|---|---|---|
| Human CD248 | 7855-CD | R&D Systems | Mouse myelom a cell line | Gln18-Arg685 | C-terminal 6-His | https://www.uniprot.org/uniprotk b/Q9HCU0/entry |

| Human RECK | 10236-RE | R&D Systems | Chinese Hamste r ovary cell line (CHO) | Gly27-Pro941 | C-terminal 6-His | https://www.uniprot.org/uniprotk b/O95980/entry |
|---|---|---|---|---|---|---|
| Human CD44 | CD4-H5226 | Aero Biosyste ms | Human HEK293 cellls | Gln21-Pro220 | C-terminal 6-His | https://www.uniprot.org/uniprotk b/P16070/entry |
| Human CSPG4 | EP885 6750 | Euprotei n | Human HEK293 cellls | Full length protein | Flag (DYKDD DDK) | https://www.uniprot.org/uniprotk b/Q6UVK1/entry |
| Human NG2 (CSPG4 ) | 2585-PG | R&D Systems | Mouse myelom a cell line | Ser1583 Ser2224 | C-terminal 6-His | https://www.uniprot.org/uniprotk b/Q6UVK1/entry |
| Human IGFBP5 | 875-B5 | R&D Systems | Mouse myelom a cell line | Leu21-Glu272 | / | https://www.uniprot.org/uniprotk b/P24593/entry |

Two different orientations were tested in the ELISA assay as summarized below:
- *Orientation* 1: the human receptor proteins were immobilized to the ELISA plate (HIS-tagged antigens: 3 µg/ml, FLAG-tagged antigens: 10 µg/ml). After blocking and washing the plate, a concentration series of biotinylated IGFBP5 was added to the plate. After washing, surface bound IGFBP5 was detected via Streptavidin-HRP.
- *Orientation 2:* the non- biotinylated IGFBP5 was immobilized to the ELISA plate (3 µg/ml). After washing and blocking of the plate, a concentration series of the human receptor proteins was added to the plate. After washing, surface bound receptors were detected via anti-HIS or anti-FLAG detection antibodies.

The binding was quantified in terms of Signal/Noise (S/N) ratio. The S/N ratio was calculated as the IGFBP5+Receptor signal versus IGFBP5+BSA signal. S/N ratios >5 were considered to be associated with significant binding.

Binding interactions between human recombinant IGFBP5 and the human receptor proteins could be detected by ELISA.

When using the assay in orientation 1, where recombinant receptors were coated on the plate, and biotinylated IGFBP5 + Streptavidin-HRP was used to detect the binding, the signal compared to the negative control (BSA) was not very high, especially for RECK, CSPG4 full length, CSPG4 fragment (i.e., NG2/MCSP) and CD44. Of note, as shown in Figure 4, panel A, a better signal was measured with CD248, that showed the highest S/N among the receptor proteins. The positive control, where the plate was coated with an anti-IGFBP5 antibody (YU384-B06), showed a strong dose-dependent signal. It is possible that in this setting, the biotin, or most of it, is masked by the IGFBP5:antigen interaction, thus lowering the binding signal.

Indeed, when testing the binding interaction with the orientation 2, where unconjugated IGFBP5 was coated on the plate, and the binding event of the antigens was detected via tags expressed on the receptor proteins, better signal intensities were registered, with CD248 showing again the highest binding and S/N ratio **(****Figure 4B****).** In this setting, all the receptors showed a significant binding with IGFBP5. Binding of human recombinant IGFBP5 to human and mouse recombinant CD248 was further confirmed in ELISA (data not shown).

According to the results obtained on IGFBP5-exposed human lung fibroblasts and to the biochemical analysis executed to assess the interaction of the recombinant proteins, CD248 has to be considered the most reliable partner of IGFBP5. These observations constitute the rationale supporting the generation of CD248-specific monoclonal antibodies, capable of inhibiting IGFBP5 binding to CD248. These antibodies can be employed to better characterize the potential role of CD248-IGFBP5 signaling in the fibrogenic process underlying fibrotic diseases, in particular IPF, and constitute a groundbreaking therapy for this unmet clinical need.

### INCORPORATION BY REFERENCE

All publications, patents, patent applications and other documents cited in this application are hereby incorporated by reference in their entireties for all purposes to the same extent as if each individual publication, patent, patent application or other document were individually indicated to be incorporated by reference for all purposes.

### EQUIVALENTS

While various specific embodiments have been illustrated and described, the above specification is not restrictive. It will be appreciated that various changes can be made without departing from the spirit and scope of the invention(s). Many variations will become apparent to those skilled in the art upon review of this specification.

## Claims

1. An inhibitor of the CD248 receptor, wherein said inhibitor inhibits or reduces the binding of IGFBP5 to said CD248 receptor, wherein said inhibitor has anti-fibrotic and/or an anti-fibrosis activity, preferably wherein said inhibitor is selected from the group consisting of:
a) a polypeptide;
b) a polynucleotide or a polynucleotide coding for said polypeptide;
c) a vector comprising or expressing said polynucleotide;
d) a host cell genetically engineered expressing said polypeptide or said polynucleotide;
e) a small molecule; and
f) a peptide, a protein, an antibody, an antisense oligonucleotide, a siRNA, antisense expression vector and a recombinant virus,
preferably wherein said inhibitor inhibits, reduces, or neutralizes the synthesis of fibronectin 1 and collagen 1 alpha 1 mRNA from human lung epithelial cells or human lung fibroblasts induced by contact with the combination of IGFBP5 and TGF-β1 and/or inhibits, reduces, or neutralizes the secretion of fibronectin (FN1) from lung epithelial cells induced by contact with the combination of IGFBP5 and TGF-β1 and/or i) reduces or inhibits extracellular matrix production and/or ii) reduces or inhibits extracellular matrix deposition and/or iii) reduces or inhibits collagen production and/or iv) reduces or inhibits fibronectin deposition in alveolar epithelial cells and lung fibroblasts.

2. The inhibitor according to claim 1 wherein said inhibitor is an isolated antibody or antigen binding fragment thereof that binds to human CD248.

3. The inhibitor according to any one of previous claims wherein said inhibitor is an isolated antibody or antigen binding fragment thereof comprising:
a heavy chain variable domain (VH) comprising:
i. a CDR1 sequence of the amino acid sequence selected from the group consisting of: SEQ ID NO: 1, 2, and 3;
ii. a CDR2 sequence of the amino acid sequence selected from the group consisting of: SEQ ID NO: 4, 5 and 6; and
iii. a CDR3 sequence of the amino acid sequence selected from the group consisting of: SEQ ID NO: 7, 8, 9, 10, 11 and 12; and/or
b. a light chain variable domain (VL) comprising:
i. a CDR1 sequence of the amino acid sequence selected from the group consisting of: SEQ ID NO: 13, 14, 15, 16, 17 and 18;
ii. a CDR2 sequence of the amino acid sequence selected from the group consisting of: SEQ ID NO: 19, 20, 21, 22 and 23; and
iii. a CDR3 sequence of the amino acid sequence selected from the group consisting of: SEQ ID NO: 24, 25, 26, 27, 28 and 29.

4. The inhibitor according to any one of previous claims wherein said inhibitor is an isolated antibody or antigen binding fragment thereof comprising:
a. a heavy chain variable domain (VH) comprising:
i. a CDR1 sequence of the amino acid sequence selected from the group consisting of a sequence as defined using abysis tool analysis (www.abysis.org) preferably as shown in Tables 8-13;
ii. a CDR2 sequence of the amino acid sequence selected from the group consisting of a sequence as defined using abysis tool analysis (www.abysis.org) preferably as shown in Tables 8-13; and
iii. a CDR3 sequence of the amino acid sequence selected from the group consisting of a sequence as defined using abysis tool analysis (www.abysis.org) preferably as shown in Tables 8-13; and/or
b. a light chain variable domain (VL) comprising:
i. a CDR1 sequence of the amino acid sequence selected from the group consisting of a sequence as defined using abysis tool analysis (www.abysis.org) preferably as shown in Tables 8-13;
ii. a CDR2 sequence of the amino acid sequence selected from the group consisting of a sequence as defined using abysis tool analysis (www.abysis.org) preferably as shown in Tables 8-13; and
iii. a CDR3 sequence of the amino acid sequence selected from the group consisting of a sequence as defined using abysis tool analysis (www.abysis.org) preferably as shown in Tables 8-13.

5. The inhibitor according to any one of previous claims wherein said inhibitor is an isolated antibody or antigen binding fragment thereof comprising as CDRs:
SEQ ID NO: 1 and SEQ ID NO: 4 and SEQ ID NO: 7 and SEQ ID NO: 13 and SEQ ID NO: 19 and SEQ ID NO: 24 or Kabat, IMGT, Chothia, AbM, or Contact CDRs of YU1022-D08 or
SEQ ID NO: 2 and SEQ ID NO: 5 and SEQ ID NO: 8 and SEQ ID NO: 14 and SEQ ID NO: 20 and SEQ ID NO: 25 or Kabat, IMGT, Chothia, AbM, or Contact CDRs of YU1023-A02 or
SEQ ID NO: 1 and SEQ ID NO: 4 and SEQ ID NO: 9 and SEQ ID NO: 15 and SEQ ID NO: 21 and SEQ ID NO: 26 or Kabat, IMGT, Chothia, AbM, or Contact CDRs of YU1023-B03 or
SEQ ID NO: 3 and SEQ ID NO: 6 and SEQ ID NO: 10 and SEQ ID NO: 16 and SEQ ID NO: 22 and SEQ ID NO: 27 or Kabat, IMGT, Chothia, AbM, or Contact CDRs of YU1023-E03 or
SEQ ID NO: 3 and SEQ ID NO: 6 and SEQ ID NO: 11 and SEQ ID NO: 17 and SEQ ID NO: 23 and SEQ ID NO: 28 or Kabat, IMGT, Chothia, AbM, or Contact CDRs of YU1024-G01 or
SEQ ID NO: 3 and SEQ ID NO: 6 and SEQ ID NO: 12 and SEQ ID NO: 18 and SEQ ID NO: 19 and SEQ ID NO: 29 or Kabat, IMGT, Chothia, AbM, or Contact CDRs of YU1025-B07,
preferably said isolated antibody or antigen binding fragment thereof comprising VH CDR1, CDR2 and CDR3 selected from Table 4 and VL CDR1, CDR2 and CDR3 selected from Table 5.

6. The inhibitor according to any one of previous claims wherein said inhibitor is an isolated antibody or antigen binding fragment thereof comprising:
a. a heavy chain variable domain sequence of the amino acid sequence selected from the group consisting of: SEQ ID NO: 30, 31, 32, 33, 34 and 35 or
b. a light chain variable domain sequence of the amino acid sequence selected from the group consisting of: SEQ ID NO: 36, 37, 38, 39, 40 and 41 or
c. the heavy chain variable domain of (a) and the light chain variable domain of (b),
preferably comprising:
a heavy chain variable domain comprising or consisting of a sequence of SEQ ID NO: 30 and a light chain variable domain comprising or consisting of a sequence of SEQ ID NO: 36;
a heavy chain variable domain comprising or consisting of a sequence of SEQ ID NO: 31 and a light chain variable domain comprising or consisting of a sequence of SEQ ID NO: 37;
a heavy chain variable domain comprising or consisting of a sequence of SEQ ID NO: 32 and a light chain variable domain comprising or consisting of a sequence of SEQ ID NO: 38;
a heavy chain variable domain comprising or consisting of a sequence of SEQ ID NO: 33 and a light chain variable domain comprising or consisting of a sequence of SEQ ID NO: 39;
a heavy chain variable domain comprising or consisting of a sequence of SEQ ID NO: 34 and a light chain variable domain comprising or consisting of a sequence of SEQ ID NO: 40; or
a heavy chain variable domain comprising or consisting of a sequence of SEQ ID NO: 35 and a light chain variable domain comprising or consisting of a sequence of SEQ ID NO: 41.

7. The inhibitor according to any one of previous claims wherein said inhibitor is an isolated antibody or antigen binding fragment thereof comprising a heavy chain variable domain encoded by a nucleotide sequence selected from the group consisting of: SEQ ID NO: 42, SEQ ID NO: 43, SEQ ID NO: 44, SEQ ID NO: 45, SEQ ID NO: 46 and SEQ ID NO:47; and/or a light chain variable domain encoded by a nucleotide sequence selected from the group consisting of: SEQ ID NO:48, SEQ ID NO: 49, SEQ ID NO: 50, SEQ ID NO: 51, SEQ ID NO: 52 and SEQ ID NO:53.

8. The inhibitor according to any one of previous claims wherein said inhibitor is an isolated antibody or antigen binding fragment thereof selected from the group consisting of: YU1022-D08, YU1023-A02, YU1023-B03, YU1023-E03, YU1024-G01 and YU1025-B07 as defined in Tables 4-18, preferably it is the antibody YU1022-D08 or YU1023-A02, or an isolated antibody or antigen binding fragment thereof that:
(a) binds specifically to an epitope on CD248, said epitope being the same or similar epitope as the epitope recognized by the monoclonal antibody YU1022-D08, YU1023-A02, YU 1023-B03, YU1023-E03, YU1024-G01 or YU1025-B07 as defined in Tables 4-18; or
(b) cross-competes for binding with the monoclonal antibody YU1022-D08, YU1023-A02, YU1023-B03, YU1023-E03, YU1024-G01 or YU1025-B07 as defined in Tables 4-18; or
(c) shows the same or similar binding affinity or specificity, or both, as any of monoclonal antibodies YU1022-D08, YU1023-A02, YU1023-B03, YU1023-E03, YU1024-G01 or YU1025-B07 as defined in Tables 4-18; or
(d) has one or more biological properties of an antibody molecule chosen from YU1022-D08, YU1023-A02, YU1023-B03, YU1023-E03, YU1024-G01 or YU1025-B07 as defined in Tables 4-18; and/or
(e) has one or more pharmacokinetic properties of an antibody molecule chosen from YU1022-D08, YU1023-A02, YU1023-B03, YU1023-E03, YU1024-G01 or YU1025-B07 as defined in Tables 4-18.

9. The inhibitor according to any one of previous claims being a human or a humanized antibody and/or being an IgG2 or IgG4 antibody, preferably an IgG2 kappa antibody, an IgG2 lambda antibody, an IgG4 kappa antibody or an IgG4 lambda antibody.

10. The inhibitor according to any one of claims 2-10 which is an isolated antibody or antigen binding fragment thereof comprising a heavy chain constant region comprising or consisting of a sequence with SEQ ID NO. 54, SEQ ID NO. 55 or SEQ ID NO. 62 and/or a light chain constant region comprising or consisting of a sequence with SEQ ID NO. 56, SEQ ID NO. 57 or SEQ ID NO. 58, preferably wherein said heavy chain constant region is a human IgG4 including a substitution at position 228, preferably a Ser to Pro substitution, and/or a substitution at position 235, preferably a Leu to Glu substitution.

11. An isolated antibody or antigen binding fragment thereof comprising:
a. a heavy chain variable domain sequence of the amino acid sequence selected from the group consisting of: SEQ ID NO: 30, 31, 32, 33, 34 and 35 or
b. a light chain variable domain sequence of the amino acid sequence selected from the group consisting of: SEQ ID NO: 36, 37, 38, 39, 40 and 41 or
c. the heavy chain variable domain of (a) and the light chain variable domain of (b).

12. An isolated polynucleotide comprising at least one sequence that encodes the inhibitor according to any one of claims 3 to 11, preferably said polynucleotide being a cDNA, preferably said polynucleotide comprising or consisting of one or more nucleotide sequences selected from the group consisting of SEQ ID NO.42 to SEQ ID NO.53.

13. A vector comprising the isolated polynucleotide of claim 12, preferably said vector being selected from the group consisting of a plasmid, a viral vector, a nonepisomal mammalian vector, an expression vector and a recombinant expression vector, or an isolated cell comprising said isolated polynucleotide of claim 12 or said vector of claim 13, preferably said isolated cell being a hybridoma or a Chinese Hamster Ovary (CHO) cell or a Human Embryonic Kidney cells (HEK293).

14. A pharmaceutical composition comprising the inhibitor according to any one of claims 1 to 11 or the polynucleotide of claim 12 or the vector or the isolated cell of claim 13 and at least one pharmaceutically acceptable carrier, preferably said composition further comprising a second therapeutic agent.

15. The inhibitor according to any one of claims 1 to 11 or the polynucleotide of claim 12 or the vector or the isolated cell of claim 13 or the pharmaceutical composition of claim 14 for use as a medicament.

16. The inhibitor according to any one of claims 1 to 11 or the polynucleotide of claim 12 or the vector or the isolated cell of claim 13 or the pharmaceutical composition of claim 14 for use in the treatment and/or prevention of fibrosis or of a fibrotic condition, preferably said fibrosis or fibrotic condition being selected from scleroderma, pulmonary fibrosis, morphea, fibrosis as a result of Graft-Versus-Host Disease (GVHD), keloid and hypertrophic scar, subepithelial fibrosis, endomyocardial fibrosis, uterine fibrosis, myelofibrosis, retroperitoneal fibrosis, nephrogenic systemic fibrosis, scarring after surgery, asthma, cirrhosis/liver fibrosis, aberrant wound healing, glomerulonephritis, and multifocal fibrosclerosis, cryptogenic fibrosing alveolitis (CFA) and idiopathic pulmonary fibrosis (IPF).
